# EUROPEAN PATENT SPECIFICATION

(11) **EP 1 578 612 B1**
(45) Date of publication and mention of the grant of the patent: **14.02.2007**
(21) Application number: 03809787.9
(22) Date of filing: 30.10.2003
(51) Int. Cl.: B41M 3/00

(54) **CONTINUOUS WEB PROCESS FOR THE MANUFACTURE OF ELECTROCHEMICAL SENSORS**
KONTINUIERLICHENBAHNVERFAHREN ZUR HERSTELLUNG ELEKTROCHEMISCHER SENSOREN
PROCEDE A BANDE CONTINUE DE FABRICATION DE CAPTEURS ELECTROCHIMIQUES

(30) Priority: 30.10.2002 US 422226 P; 30.10.2002 US 422230 P; 27.12.2002 US 436683 P; 27.12.2002 US 436685 P; 31.01.2003 US 443930 P
(43) Date of publication of application: 28.09.2005
(73) Proprietor: Lifescan Scotland Ltd, Inverness IV2 3ED (GB)
(72) Inventor: DAVIES, Oliver William Hardwicke, Croy, Inverness IV2 5PG (GB); ARMSTRONG, Malcolm, Gordon, Inverness IV2 4WS (GB); MARSHALL, Robert, Inverness IV2 5FX (GB); MITCHELL, Darren, Iain, Inverness IV2 3BF (GB); O'REILLY, Thomas Joseph, Farr, Inverness IV2 6AW (GB); ROBERTSON, Emma-Louise, Culloden, Inverness IV2 7TL (GB); SIM, Andrew Graham, Broadford, Skye IV49 9BL (GB); YEUDALL, Robert Malcolm, Milton o f Leys, Inverness IV2 6HF (GB)
(74) Representative: Tunstall, Christopher Stephen
(86) International application number: PCT/GB2003/004689
(87) International publication number: WO 2004/039897

(56) References cited:
- WO-A-98/43075
- US-A- 6 103 033

## Description

### BACKGROUND OF THE INVENTION

### Field of the Invention

The present invention relates, in general, to a continuous web manufacturing process for electrochemical sensors.

### SUMMARY OF THE INVENTION

An exemplary embodiment of a method for manufacturing an electrochemical sensor according to the present invention includes transporting a substrate web past at least one print station and printing at least one electrochemical sensor electrode on the substrate web at the print station(s). The printing is accomplished by applying an ink composition to substrate web. The ink composition which is applied includes graphite, carbon black, a resin and at least one solvent. In addition, a weight ratio of graphite to carbon black in the ink composition is in a range of from 4:1 to 1:4 and a weight ratio of a sum of graphite and carbon black to resin is in a range of from 10:1 1 to 1:1.
Methods for making chemical sensors are described in WO-A-98/43075 and in US-A-6 103 033

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a schematic diagram depicting 8 sections of the web printing process.

FIG. 2A is a schematic diagram depicting a first and second sections of the web printing process.

FIG. 2B is a schematic diagram depicting a third, fourth, and fifth sections of the web printing process.

FIG. 2C is a schematic diagram depicting a sixth and seventh sections of the web printing process.

FIG. 3 is a schematic diagram depicting a humid environment around a fifth and sixth sections of the web printing.

FIG. 4 is a bottom view depicting a humid environment around a fifth and sixth sections of the web printing.

FIG. 5 is a perspective view of a pipe with perforations.

FIG. 6 is a schematic diagram depicting a flood cycle.

FIG. 7 is a schematic diagram depicting a print cycle.

FIG. 8 is a schematic diagram depicting 2 different squeegee angles.

FIG. 9 is a schematic diagram depicting 2 different squeegee positions.

FIG. 10 is a schematic diagram depicting a screen snap distance.

FIG. 11 is an exploded view of a preconditioning zone (211).

FIG. 12 is an exploded view of the first drying zone (217).

FIG. 13 is an exploded view of a second drying zone (224).

FIG. 14 is an exploded view of a third drying zone (230).

FIG. 15 is an exploded view of a fourth drying zone (236).

FIG. 16 is an exploded view of a first cleaning unit (204).

FIGs. 17A-17D are views of an insulation layer to carbon layer with proper registration.

FIGs. 18A-18D are views of an insulation layer to carbon layer with improper registration when the artwork resulting from the screen 301 is stretched.

FIGs. 19A-19D are views of an insulation layer to carbon layer with improper registration when the art work from screen 301 has not stretched.

FIGs. 20A-20D are schematic diagrams depicting the print results for operator registration of the web using a first view guide for visual inspection during an initial registration process.

FIG. 21A is an example of a sensor sheet with a first and second web view guides; first, second, third and fourth Y registration marks; and X registration marks.

FIG. 21B is an exploded view of one row within a sensor sheet with a carbon X registration mark.

FIG. 21C is an exploded view of one row within a sensor sheet with an insulation X registration mark over coating a carbon X registration mark.

FIG. 22 is a schematic diagram of parameters X, Y, and θ used to register the web printing process.

FIG. 23 is a flow chart illustrating a sequence of steps in a process according to an exemplary embodiment of the present invention.

### DETAILED DESCRIPTION OF ILLUSTRATIVE EMBODIMENTS OF THE INVENTION

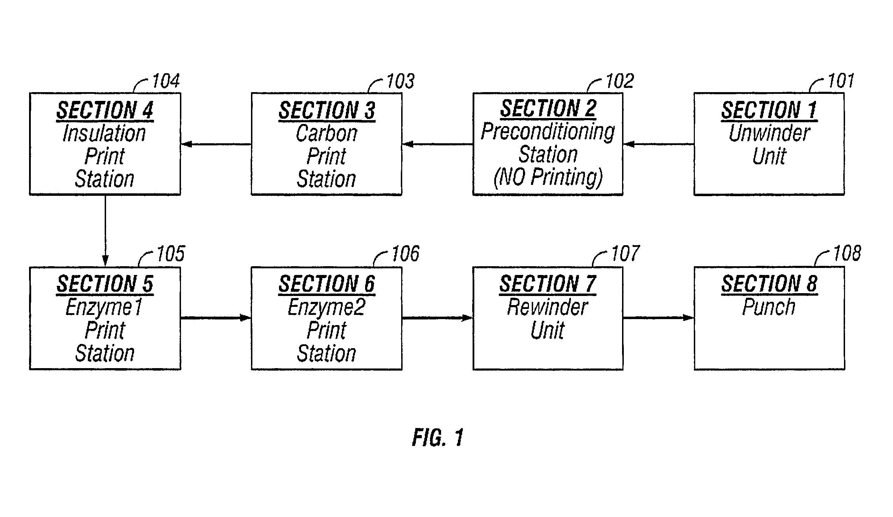

FIG. 1 is a schematic diagram depicting 8 sections of the web printing process according to the present invention. Section 1 is an unwinder unit 101. Section 2 is a pre-conditioning station 102. Section 3 is a carbon print station 103. Section 4 is an insulation print station 104. Section 5 is a first enzyme print station 105. Section 6 is a second enzyme print station 106. Section 7 is a rewinder unit 107. Section 8 is a punch 108. It will be understood by those skilled in the art that while the following description relates to a process and apparatus concerning these S sections, the process and apparatus of the invention can be embodied in greater or fewer numbers of sections. For example while 4 print stations are envisaged in this embodiment, one or more print stations could be used without departing from the scope of the invention. In one embodiment there are a minimum of two print stations for printing an electrode layer and a reagent layer.

In one embodiment of the present invention, Section 1 may be implemented using a substrate material unwind unit 101 such as, for example, a Martin Unwinder/Automatic Splice which is available from Martin Automatic Inc. in Rockford, IL. In this embodiment of the invention, Sections 2, 3, 4, 5 and 6, may be implemented using a modified Kammann Printer, which is available from Werner Kammann Maschinefabrik Gmbh, model number 4.61.35, in Bünde, Germany. In this embodiment of the invention, Section 2 may be pre-conditioning unit 102. Pre-conditioning unit 102 may be used to precondition substrate 242 prior to printing and sections 3, 4, 5 and 6 may be used to screen print carbon, insulation, first enzyme and second enzyme inks onto a substrate 242. Section 7 may include rewinder unit 107 such as, for example, a Martin Rewinder, which is available from Martin Automatic Inc. in Rockford, IL. Section 8 may include a punch 108 such as, for example, a Preco punch which is available from Preco Press, in Lenexa, Kansas as model number 2024-P-40T XYT CCD CE. While specific models of apparatus are mentioned, these pieces of apparatus may be varied and/or replaced and/or omitted altogether without departing from the scope of the invention as will be understood by those skilled in the art.

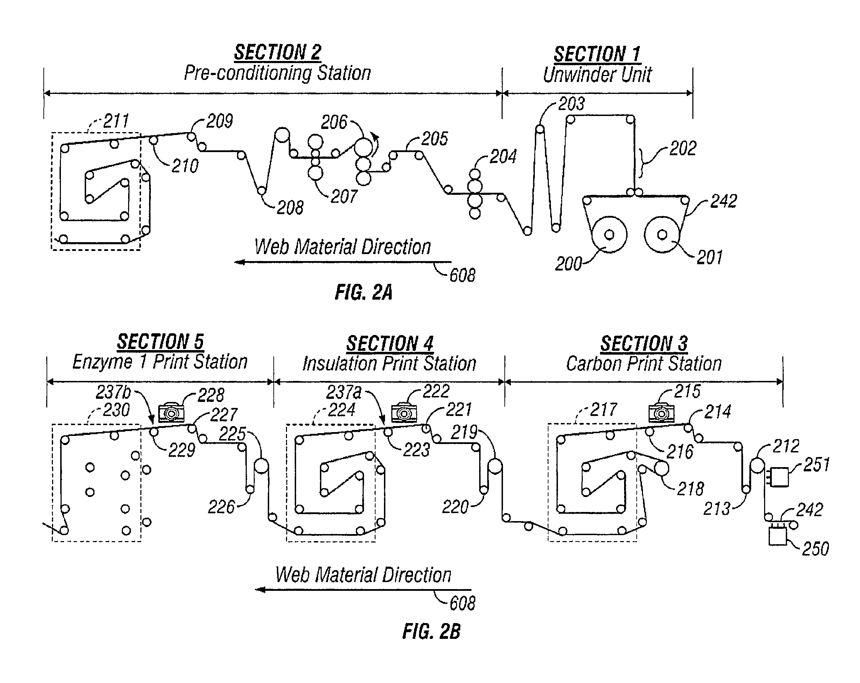

FIGs. 2A, 2B and 2C are schematic diagrams illustrating the path of substrate 242 as it passes through Sections 1-8 of a web printing process according to the present invention. In one embodiment of the Invention, the material used for substrate 242 may be a polyester material (trade name Melinex ® ST328), which is manufactured by DuPont Teijin Films. Substrate 242 is supplied in a roll of material, which may be, for example, nominally 350 microns thick by 370mm wide and approx. 660m in length. These dimensions of thickness and width have been found to be particularly suitable for the production of electrochemical sensors by flat screen printing on a web of substrate. This is because of the requirement for the material to be robust for printing yet manipulable through the apparatus and of sufficient width to accommodate a suitable quantity of sensors to render the process commercially viable. Substrate 242 may include an acrylic coating applied to one or both sides to improve ink adhesion. Polyester is a preferred material because it behaves satisfactorily at elevated temperatures and tensions used during the web process according to the present invention. While polyester and indeed Melinex are the preferred materials in one embodiment of the invention, the use of other materials can be envisaged by those skilled in the art from the description provided herein. Indeed, amongst other things, variations in material thickness, width and length can be envisaged, a larger width or length offering additional capacity for the production of sensors and a variation in material thickness in some circumstances aiding the preconditioning, or registration during printing. In a preferred embodiment of the present invention, prior to entering carbon print station 103, substrate 242 is exposed to a heat stabilization process, by heating the substrate up to 185 °C without placing it under significant tension to try and ensure that substrate 242 experiences minimum dimensional distortion during the web printing process where temperatures of between 140 and 160°C at tensions up to 165 N may be encountered. Typically the tension used has been minimal, just to sufficient to drive the web through the heater. However, it has been found that despite this heat stabilization process, variations in registration from print step to print step can occur causing sensor failure. Thus, a preconditioning step has been introduced immediately prior to printing. As will be explained hereinafter, in the preconditioning step (section 1) the substrate is heated to a temperature (typically 160°C) greater than any temperature it encounters during the later printing steps. In one preferred embodiment the substrate is also kept under tension (typically around 165N) during this preconditioning step. Indeed in this embodiment, the combination of preconditioning and placing under tension has greatly reduced the variations in print registration and improved the resultant product yield. In one embodiment of the invention, rolls of substrate 242 are spliced together in either unwinder unit 101 or rewinder unit 107 using splicing tape such as, for example, PS-1 Splicing Flat-back Paper Tape from Intertape Polymer Group.

FIG. 2A is a schematic diagram depicting section 1 and section 2 of a web printing process according to one embodiment of the present invention. In FIG. 2A, section 1 is an unwinder unit 101. Unwinder unit 101 includes first arbor 200, second arbor 201, first splice unit 202, and first accumulator 203. In FIG. 2A, section 2 is pre-conditioning Station 102. Pre-conditioning Station 102 includes first cleaning unit 204, second splice unit 205 which typically is not used, inbound nip roller 206, second cleaning unit 207, load cell 208, first print roller 209, first drive roller 210 and first drier zone 211.

In the embodiment of the invention illustrated in FIG. 2A, unwinder unit 101 consists of, for example, a Martin Unwinder/Automatic Splice which is used to facilitate the continuous movement of substrate 242 into pre-conditioning station 102 under a tension of approximately 80N. Unwinder unit 101 may include a first unwind arbor 200 and a second unwind arbor 201. Note that an arbor can also be referred to as a mandrel. First unwind arbor 200 holds a roll of substrate material 242 and continuously feeds substrate 242 into pre-conditioning station 102 of section 2. Second unwind arbor 201 holds a standby roll of substrate 242, which is automatically spliced to the end of the roll of substrate 242 from first unwind arbor 200 ensuring a semi-continuous supply of substrate 242. This continuous process repeats from first unwind arbor 200 to second unwind arbor 201. A substrate material accumulator 203 stores a predetermined length of substrate 242 and dispenses the stored substrate 242 into pre-conditioning station 102 of section 2 while the splicing operation takes place in first splice unit 202 (during which time both the first unwind arbor 200 and second unwind arbor 201 are stationary). The splice created is a butt splice with a length of splice tape on either side of the material at the joint. In order to ensure quality, approximately 10m of printed substrate may be discarded either side of the splice. First unwind arbor 200 and second unwind arbor 201 includes web edge guides (not shown) which guide substrate 242 into first splice unit 202. The web edge guides are adapted to prevent substrate 242 from wandering as it is being fed into first splice unit 202.

Typically the machine used in the invention is set up to produce between 2 and 10 and more usually 6 rolls of substrate at any one time. For those print stations connected to a continuous supply of ink, the number of rolls to be used is not usually a problem. However, for the two enzyme print stations, to which a limited amount of ink is supplied, the number of rolls to be used is an important input parameter. Indeed the number of rolls to be used determines the amount of ink placed on the screen prior to start of the printing process. For example for a 6 roll run, 6 (or rather just more than 6) rolls worth of enzyme ink are placed on the screen prior to the start of printing in each of sections 5 and 6. Thus, the enzyme ink needs to be kept in readiness for printing throughout the print run to ensure consistent printing of enzyme over the whole life of the print run. A wall has been placed about the screen in the enzyme print stations to ensure that a sufficient amount of enzyme ink can be added to the screen without requiring the screen to be topped up during a run and also reducing the risk of the enzyme ink overflowing the screen and onto the web substrate running below it.)

In one embodiment of the present invention, substrate 242 is held under a tension of approximately 165N throughout the process in order to maintain registration of the four layers to be printed (typically the print registration tolerance is 300 µm). The substrate 242 is also subjected to various temperatures of 140 °C or less in order to dry the printed inks during each printing step. Due to this tension and temperature, there may be a tendency for substrate 242 to stretch or expand during the process and consequently fall outside the registration tolerance. Indeed the image size variation from print stage to print stage and print run to print run as well as within the print run itself was unpredictable and higher than could be tolerated.

In the embodiment of the invention illustrated in FIG. 2A, section 2 is a pre-conditioning station 102. Pre-conditioning occurs before any image is printed onto the substrate. Substrate 242 is pre-conditioned to reduce the amount of expansion and stretch within subsequent sections of the web process and also to aid the registration of substrate 242 through sections 3-6. Preconditioning station can heat substrate 242 to a temperature, which is not exceeded in the subsequent print steps. Typically this takes place under tension of between 150 and 180N more typically around 165N. However, in another embodiment pre-conditioning station 102 can heat substrate 242 to a temperature sufficient to remove the irreversible stretch from substrate 242, again optionally while under tension as described above.

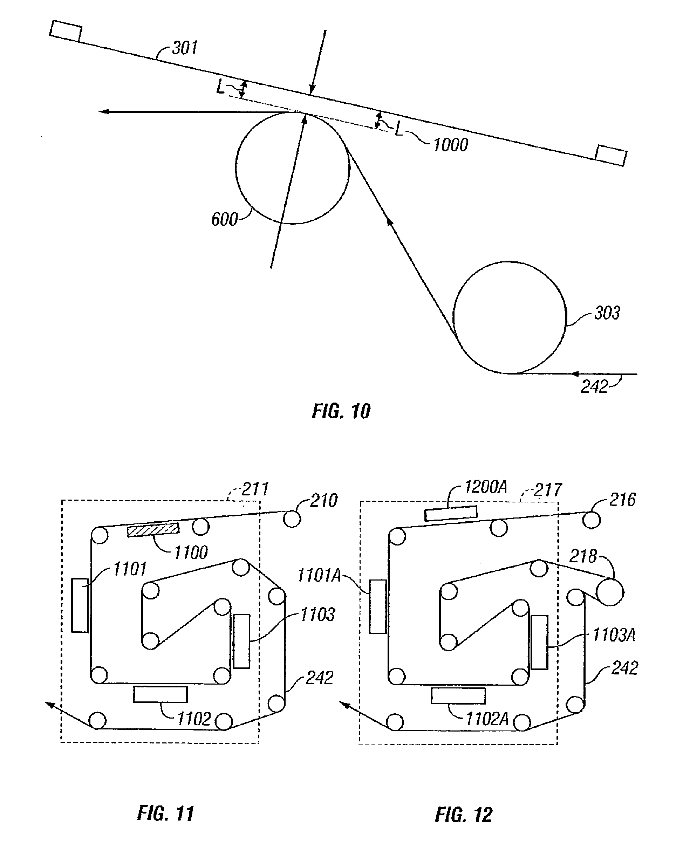

In one embodiment of the invention, the substrate is heated to approximately 160 °C in the preconditioning zone 211, which is illustrated in more detail in FIG. 11. As explained above, in one embodiment of the present invention, the temperature to which substrate 242 is heated in pre-conditioning station 102 is not met or exceeded during subsequent processing of substrate 242, including subsequent drying steps. Subsequent print processes may compensate for the slightly larger image due to stretching caused by the process of pre-conditioning station 102 by the provision of a slightly larger stencil screen size (typically 750µm in the direction of travel of the web). The provision of new screens can be problematical. Other parameters can therefore be varied at each print station to accommodate a variation in image size without replacing the screen, such as the relative speed of the screen and the web. Nevertheless, there is a limit to the amount of image size variation that can be accommodated. It is therefore preferable to precondition the substrate as described herein reducing the overall image size increase and reducing the variation in said image size increase.

In one embodiment of the present invention, pre-conditioning station 102 also includes additional elements, which perform functions, which facilitate proper operation of a web manufacturing process according to the present invention. In pre-conditioning unit 102, there are two web cleaning units, a first cleaning unit 204 and a second cleaning unit 207 which clean the top and underside of substrate 242. First cleaning unit 204 and second cleaning unit 207 use tacky adhesive coated rollers to remove particulates from substrate 242 prior to any printing step. First cleaning unit 204 may be, for example, a cleaner commercially available from KSM Web Cleaners, model number WASP400, in Glasgow, United Kingdom. Second cleaning unit 207, for example, a cleaner commercially available from Teknek. Pre-conditioning station 102 further includes inbound nip roller 206 and a load cell 208. Inbound nip roller 206 is used to control the tension of substrate 242 (specifically the tension between inbound nip roller 206 and outbound nip roller 238). Inbound nip roller 206 is linked via a control system (not shown) to load cell 208. Substrate 242 is removed from second enzyme print station 106 in section 6 at a constant rate by outbound nip roller 238. Load cell 208 in Section 2 measures the tension of substrate 242 when it is moving through the web process according to the present invention. Inbound nip roller 206 adjusts its speed in order to control the tension at a predetermined set point. A typical substrate tension in a web manufacturing process according to the present invention would be approximately 150N to 180N and more specifically 160N to 170N, in this embodiment the tension is approximately 165N.

FIG. 2B is a schematic diagram depicting section 3, section 4 and section 5 of a web printing process according to the present invention. In FIG. 2B, section 3 is carbon print station 103. Prior to printing (a cleaning system is installed (available from Meech), which cleans the top side (print side) and underside of the substrate using a vacuum and brush system, the top brush and vacuum station 251 and bottom brush and vacuum station 250 are offset to one another. The top brush and vacuum station 250, contacts the substrate immediately prior to the chilled roller 212 and accumulator 213 and is the closest accessible point prior to carbon printing. The underside brush and vacuum station 251, contacts the substrate immediately after the substrate exits the pre-conditioning unit 102. Carbon print station 103 includes first chilled roller 212, second accumulator 213, second print roller 214, first vision sensor 215, second drive roller 216, first drier zone 217 and second chilled roller 218. In the embodiment of the invention illustrated in FIG. 2B, section 4 is insulation print station 104. Insulation print station 104 includes third chilled roller 219, third accumulator 220, third print roller 221, second vision sensor 222, first Y registration system (not shown) at position 237A, third drive roller 223 and second drier zone 224. In FIG. 2B, section 5 is first enzyme print station 105. First enzyme print station 105 includes fourth chilled roller 225, fourth accumulator 226, fourth print roller 227, third vision sensor 228, second Y registration system, at 237B (not shown), fourth drive roller 229 and third drier zone 230.

In a process according to the present invention, section 3 of the web manufacturing process is where carbon printing takes place. Of course, as will be appreciated by those skilled in the art, the number and type of printing processes can be varied without departing from the invention in its broadest context. For example, two carbon prints may be provided or one or more prints with carbon with metallic particles, silver/silver chloride ink or gold or palladium based inks may be used to provide an electrode layer in the electrochemical sensors. The insulation and reagent layers may also be varied in their composition, order of deposition, thickness of deposition and layout as well as in other parameters apparent to those skilled in the art from the embodiments described herein. In section 3, the carbon artwork for the electrochemical sensors manufactured in accordance with the present invention may be printed utilizing screen-printing. The basic components of the carbon print station 103 are illustrated in FIG. s 6 and 7. In particular, a suitable print station according to the present invention includes a screen 301, lower print roller 303, print roller 600, a flood blade 603, a squeegee holder 605 and a squeegee 606. In carbon print station 103, print roller 600 is second print roller 214. Screen 301 is of generally flat construction and typically comprises a mesh arranged to provide a negative of the artwork desired. Carbon ink is applied to the mesh and pushed through it during printing. At this stage the flat screen may be deformed slightly out of a flat shape by the weight of the ink (this is especially true for the enzyme print steps in which all of the ink to be used during the entire print run is usually deposited on the screen at the start of the print run) and the pressure from the squeegee pushing the ink through the mesh stencil.

In a flood cycle process in accordance with the present invention, screen 301 is charged with ink 604 by moving squeegee 606, flood blade 603, print roller 600, and lower print roller 303, in first direction 608 which corresponds to the web movement of substrate 242. Screen 301 is moved in second direction 607 opposite to first direction 608 of substrate 242 for the flood cycle where ink 604 is charged onto screen 301.

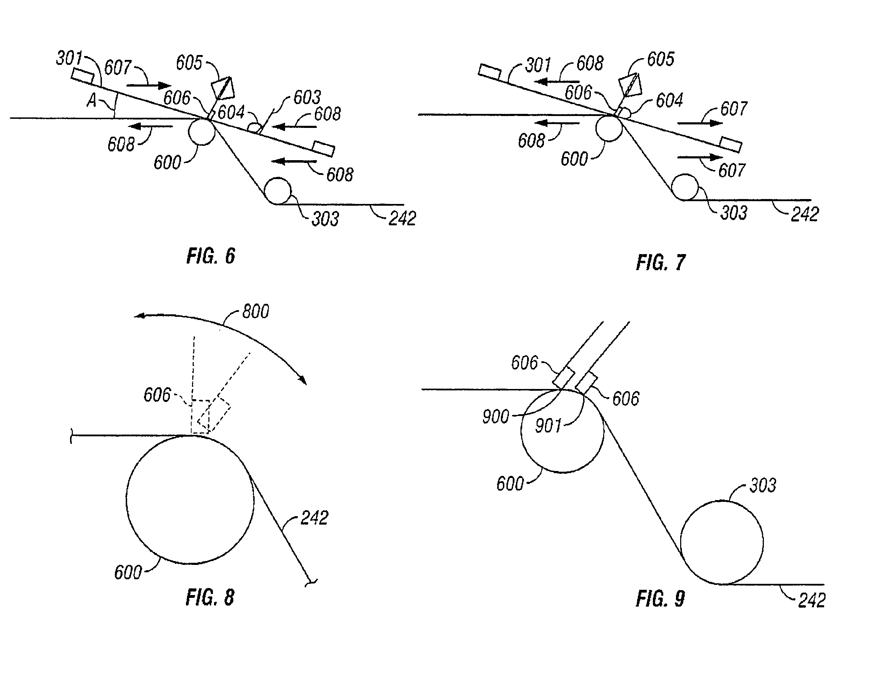

In a subsequent print cycle process in accordance with the present invention, as illustrated in FIG. 7, squeegee 606 transfers ink 604 through the screen 301 and onto substrate 242. During the print cycle, the squeegee 606, flood blade 603, print roller 600, and lower print roller 303 all move in second direction 607 which is opposite to the web movement of substrate 242. Screen 301 is moved in first direction 608 which corresponds to the web movement of substrate 242 for the print cycle where ink 604 is pushed through screen 301 and deposited on substrate 242. Thus during the print cycle the screen 301 moves in the same direction as the web substrate at the same or very nearly the same speed as the substrate. The screen 301 is substantially flat when at rest although in use it is pushed by the squeegee 606 towards the web becoming slightly distorted as this happens and substantially returning to it's original shape once the squeegee 606 is removed. The screen 301 then moves in the opposite direction to the substrate as it is reloaded with ink 604 ready for the next print cycle. When the ink is loaded onto the screen 301 the weight of the ink may ever so slightly bend the screen. The screen 301 is at an angle to the direction of travel 608 of the web as it leaves the print station. This arrangement (the angle being typically around 10 to 30 degrees and more specifically around 15 degrees) improves ink release from the screen onto the substrate improving print definition and reproducibility. The screen to substrate angle, squeegee angle, screen to squeegee distance, squeegee to print roller position, snap distance, relative speeds of substrate and screen and squeegee pressure can all be used to control and optimize the resultant print definition and consistency across a card (One embodiment of a screen printing mechanism is described in more detail in issued U.S. Patent No. 4,245,554

In particular, in carbon print station 103, the ink in question is a carbon ink. An example of a suitable carbon ink is set forth herein below. In this embodiment of the current invention, screen 301 is flooded with ink 604 prior to using squeegee 606 to transfer the ink 604 through the screen and onto substrate 242. The printed carbon artwork deposited on substrate 242 is then dried using, for example, hot air at 140 °C directed onto the printed surface of the substrate using four separate drying banks within the first drier zone 217, which is illustrated in more detail in FIG. 12.

Once apprised of the present disclosure and the disclosure of provisional patent application No. 60/436,683, one skilled in art will recognize that a variety of ink compositions (also referred to as inks or carbon inks) can be utilized in processes for manufacturing electrochemical sensors (e.g., web-based processes according to the aforementioned provisional patent application). However, ink compositions used in the present invention are based on the recognition that it is particularly desirable to employ ink compositions that (i) provide for a printed electrode of a manufactured electrochemical sensor to possess beneficial electrochemical and physical characteristics (such as, for example, electrochemical characteristics that are essentially equivalent to those provided by a batch manufacturing process and/or a desirable overpotential, electrochemical surface area, resistance, capacitance, and stability) and (ii) is compatible with relatively high-speed continuous web processing techniques.

For an ink composition to be compatible with high-speed continuous web processing techniques, the ink composition should be dryable in a drying duration (time) that does not limit the speed of the continuous web process (e.g., a short drying duration in the range of 30 seconds to 60 seconds). Such a short drying duration requires more severe (harsher) drying conditions (e.g., the use of 140 °C air at a velocity of 60 m³/minute) than a conventional batch process. Unfortunately, when such severe drying conditions are used, there is a tendency for the surface of conventional ink compositions to bum and/or for a portion of a conventional ink composition that is in contact with a substrate to remain undried. Furthermore, the combination of severe drying conditions and conventional ink compositions can result in the formation of an electrode (e.g., a carbon electrode) with undesirable electrochemical characteristics. Therefore, conventional ink compositions typically require the use of relatively slow drying conditions and a relatively long drying duration (e.g., approximately 15 or more minutes).

It has been unexpectedly determined that ink compositions used in the present invention, which include graphite, carbon black, a resin and one or more organic solvents, are particularly useful in the manufacturing of electrochemical sensors. Ink compositions used in the present invention provide for a printed electrode of a manufactured electrochemical sensor to possess beneficial electrochemical and physical characteristics. The ink compositions are also compatible with relatively high-speed continuous web processing techniques. This compatibility is due to the relatively high conductivity of the ink compositions, which enables a thinner printed film (i.e., printed electrode). In addition, it is postulated without being bound that the printed electrode is easily dried due to its thin nature and the use of an ink composition that includes at least one solvent of an appropriate boiling point.

The graphite, carbon black and resin percentages of ink compositions used in the present invention are predetermined such that a weight ratio of graphite to carbon black is in the range from 4:1 to 1:4 and a weight ratio of the sum of graphite and carbon black to resin is in the range of from 10:1 1 to 1:1. Factors which can influence optimization within of the aforementioned ratios are the resulting electrochemical surface area, overpotential for oxidizing a redox mediator, as well as the stability, resistance, and capacitance of a printed carbon film (e.g., carbon electrode).

It is envisioned that ink compositions used in the present invention can be used to manufacture carbon films that serve as electrochemical sensor electrodes. Such carbon films can be used in an electrochemical glucose biosensor, wherein a current is measured at a constant potential and the magnitude of the measured current is indicative of a glucose concentration. The resulting current can be linearly calibrated to output an accurate glucose concentration. A method of calibrating electrochemical glucose biosensors is to define multiple calibration codes within a calibration space, in which a particular calibration code is associated with a discrete slope and intercept pair. For a particular lot of electrochemical sensors, a measured current output may be mathematically transformed into an accurate glucose concentration by subtracting an intercept value from the measured current output and then dividing by the slope value.

It should be noted that the measured current output, slope and intercept values can be influenced by the electrochemical surface area, overpotential for oxidizing a redox mediator, as well as the stability, resistance, and capacitance of the carbon film that serves as the electrochemical sensor electrode. Therefore, the weight ratio of graphite to carbon black and weight ratio of the sum of graphite and carbon black to resin can be optimized to provide a desired range of slopes and intercepts.

Any suitable graphite and carbon black known to one skilled in the art can be employed in ink compositions used in the present invention. In this regard, a carbon black with a surface area of, for example, 20 to 1000 m²/g is generally suitable in terms of providing a requisite conductivity. In general, the conductivity of the carbon black increases with the its surface area and a relatively high conductivity carbon black can be beneficial in terms of providing desirable electrochemical characteristics. Other characteristics of carbon black that are desirable for use in the present invention are high conductivity, low sulfur content, low ionic contamination and easy dispersability. Suitable carbon blacks include, but are not limited to, Vulcan XC-72 carbon black (available from Cabot) and Conductex 975B carbon black (available from Sevalco). Other types of carbon of carbon black that may be suitable for the present invention are Black Pearls (available from Cabot), Elftex (available from Cabot), Mogul (available from Cabot), Monarch (available from Cabot), Emperor (available from Cabot), Regal (available from Cabot), United (available from Cabot), and Sterling (available from Cabot), Ketjen Black International Company (available from Ketjen Black), Mitsubishi Conductive Carbon Black (available from Mitsubishi Chemical), Shawinigan Black (available from Chevron Phillips Chemical Company LP) and Conductex® (available from Columbian Chemicals Company). Suitable graphites include, but are not limited to, Timex KS15 carbon (available from G&S Inorganic). The particle size of graphite can be, for example, between 5 and 500 µm, but more preferably can be 15 µm. Other types of graphite that may be suitable for the present invention are Timrex KS6 to Timrex KS500 where the number following the term KS represent the particle size in units of microns. Other characteristics of graphite that are desirable for use in the present invention are high conductivity, low ash content, low sulfur content and low inorganic impurities.

In general, the surface area of graphite is much less than the surface area of carbon black by virtue of graphite's non-porous nature. For example, the surface area of Timrex KS 15 is approximately 12 m²/g. It is theorized without being bound that the use of graphite in ink compositions according to the present invention enhances the electron transfer properties of electrodes manufactured using the ink compositions. However, an optimized weight percentage of carbon black is needed in the ink composition in order to increase the overall conductivity of the ink composition. Otherwise, the use of graphite alone would result in a film having a very high electrode resistance.

The electrochemical surface area of a carbon electrode may represent the portion of the carbon electrode that can contribute to the oxidation of mediator. Graphite, resin and carbon black can have varying degrees of conductivity and, thus, influence the proportion of the geometric electrode area that can participate in the oxidation of a mediator. The geometric electrode area represents the area of a carbon electrode that is exposed to a liquid sample. Since the electrode material (i.e., an ink composition used to manufacture an electrode) can have an insulating resin therein, the electrochemical area may be smaller than the geometric area. In general, the current output of a glucose biosensor is directly proportional to the electrochemical surface area. Therefore, variations in the electrochemical surface area may influence the slope and intercept of the glucose biosensor.

The stability of a carbon electrode is important in designing robust glucose biosensors which are useful to diabetic users. In general, stability of a carbon electrode can be optimized by choosing an appropriate resin and ensuring that sufficient solvent is removed from the carbon electrode during drying. It is possible that insufficiently dried carbon electrode can outgas solvent during its storage and thus cause a change in the performance of the resulting glucose biosensor. Furthermore, the stability of the carbon electrode may influence the slope and intercept of the glucose biosensor.

The resistance and capacitance are intrinsic properties of a carbon electrode and are strongly dependent of the proportions of carbon black, graphite, and resin within the carbon electrode. For example, the resistance of a carbon electrode will increase when a higher proportion of resin or graphite is used in the electrode's formulation. The resistance of an electrode may influence the electrochemical current of a glucose biosensor because of the uncompensated IR drop between a reference electrode and a working electrode. The capacitance of an electrode will depend on the ability of an ionic double layer to form at an electrode/liquid interface. The formation of such an ionic double layer will influence the magnitude of the measured current. Certain proportions of carbon black, graphite, and resin are likely to enhance the ability of the ionic double layer to form. Therefore, the resistance and capacitance of a carbon electrode can influence the slope and intercept of a glucose biosensor.

With respect to an electrochemical sensor of a glucose measuring system that includes a working electrode, it is desirable that a relatively low potential be applied to the sensor's working electrode in order to minimize the effect of oxidizable interferences that are often endogenous to physiological samples. To achieve such a relatively low potential, it is beneficial that the material from which the working electrode is formed enables the oxidation of ferrocyanide (or other redox mediator) at the lowest possible potential. This can be achieved, for example, by minimizing the activation energy required for electron transfer between the working electrode and ferrocyanide (or other redox mediator). In this regard, it has been determined that the ratio of graphite to carbon black is critical in defining (e.g., minimizing) the overpotential required for the oxidation of a reduced redox mediator such as, for example, ferrocyanide by an electrode of the electrochemical sensor.

For the above reason, ink compositions used in the present invention have a ratio of graphite to carbon black that is in the range of from 4:1 to 1:4. Furthermore, a particularly beneficial ratio of graphite to carbon black in terms of defining the overpotential has been determined to be 2.62:1. It has also been determined that the ratio of the sum of graphite and carbon black to resin also influences the overpotential for oxidizing reduced redox mediator such as, for example, ferrocyanide. And it is for this reason that the ratio of the sum of graphite and carbon black to resin is in a range of from 10:1 1 to 1:1, with a particularly beneficial ratio being 2.9:1.

The resin employed in ink compositions used in the present invention can be any suitable resin known to one skilled in the art including, but not limited to, terpolymers that comprise vinyl chloride, vinyl acetate and vinyl alcohol. One such terpolymer is VAGH resin available from Union Carbide. Resin is employed in the ink composition as a binding agent and to help adhere carbon black and graphite to a substrate (such as web substrate) during the manufacturing of an electrochemical sensor. Additionally, resins such as VAGH will provide flexibility to the printed film, which is especially useful in a continuous web based processes where printed films must be stable when rewound into a roll format.

The at least one solvent that is included in ink compositions used in the present invention is a solvent in which the resin is soluble and which has, for example, a boiling point in the range of 120 °C to 250 °C. It is desirable that the boiling point not be less than 120 °C in order to insure that rapid bubbling does not occur in a printed ink composition film when the film is exposed to a drying temperature of 140 °C. Such rapid bubbling during the drying process could cause the printed films (i.e., printed electrodes) to have a rough surface which may be undesirable. If a solvent's boiling point is greater than 250 °C, there is a risk that the ink composition will not sufficiently dry when exposed to, for example, a drying temperature of 140 °C and an air flow of 60 m³/min for a duration in the range of approximately 30 seconds to 60 seconds.

Suitable solvents include, for example, a combination of methoxy propoxy propanol (bis-(2-methoxypropyl) ether), isophorone (3,5,5-trimethyl-2-cyclohenex-1-one) and diacetone alcohol (4-hydroxy-4-methyl-2-pentanone). It should be noted that a combination of at least two solvents can be particularly beneficial because of a possible decrease in boiling point of the aggregate solvent mixture, i.e., azeotrope mixture. The use of isophorone alone can provide a carbon ink composition with favorable electrical properties. However, the combination of isophorone with methoxy propoxy propanol and diacetone alcohol can accelerate the drying of the carbon ink. Once apprised of the present disclosure, one of skill in the art can choose other suitable solvents with drying properties that are appropriate to various drying conditions.

Ink compositions used in the present invention have several beneficial properties including being fast-drying while providing for the manufacturing of an electrode with desirable physical and electrochemical properties. The ink compositions can be dried quickly using relatively severe conditions and are, therefore, compatible with high-speed continuous web-based processing techniques. In addition, the ink compositions also enable the manufacturing of highly conductive carbon electrodes even when a relatively thin coating (e.g., a coating with a thickness in the range of 5 µm to 20 µm, for example 10 µm) of the ink composition is employed. Furthermore, the ink compositions are of low toxicity, bind well to substrate layers (and to insulating layers), possess a good print quality and long screen life (i.e., the ink composition does not solidify when used for a long period in screen printing), and are of low cost.

Suitable conductive ink that can be used, include, but is not limited to, carbon with metallic particles, silver/silver chloride, gold based, palladium based conductive printable inks.

Ink compositions used in the present invention can be prepared using any suitable ink preparation technique, including techniques that are well known to those of skill in the art. In one embodiment of the invention, the weight % of solids is in the range of 36 to 44% and the weight % of solvent is in the range of 56 to 64%. One factor which helps control the quality and thickness of an ink composition is viscosity. It should be noted that the weight % of solids influences the viscosity of the ink. In one embodiment of the current invention, the ink composition has a viscosity between 11 to 25 Pascal seconds at 50 RPM, and between 21 to 43 Pascal seconds at 10 RPM (25 °C). Experimentally, it was found that inks with a weight % of solids in the range of 36% to 44% resulted in glucose biosensors having a relatively constant calibration slope when preparing glucose biosensors using such inks (see graph below). It is possible that the more robust calibration slopes was a result of a more uniform electrode thickness resulting from the optimized viscosity.

Carbon ink can be made, for example, by first dissolving 9.65 g of VAGH in an organic solvent made up of 46.53 g of methoxy propoxy propanol, 7.90 g of isophorone and 7.89 g of diacetone alcohol in a closed vessel. Next, 7.74 g of carbon black is added to the mixture and then mixed in the closed vessel. 20.29 g of graphite is then added to the mixture, followed by mixing in the closed vessel. In order to ensure sufficient homogenization, a triple roll milling is performed on the mixture followed by more mixing.

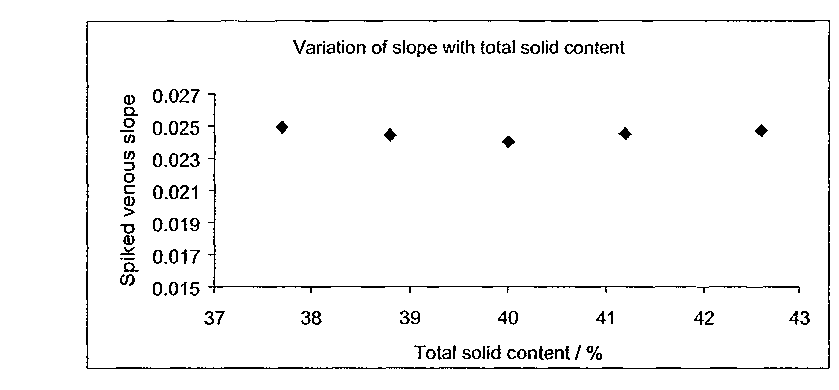

Another embodime nt of an ink composition for use in manufacturing electrochemical sensors according to the present invention includes (i) between approximately 17 and 21 % by weight of graphite; (ii) between approximately 6.5 and 8.0% by weight of carbon black; (iii) between approximately 12.4 to 15.2% by weight of a terpolymer resin that includes vinyl chloride, vinyl acetate and vinyl alcohol; and (iv) between approximately 55.8 to 64.1% by weight of a solvent mixture that includes isophorone, diacetone alcohol and methoxy propoxy propanol.

The ink composition can be employed in the manufacturing of electrochemical sensors by a variety of processes including, but not limited to, those described in Provisional Patent Application No. 60/436,683. In this regard and referring to FIG. 23, a process 2300 for manufacturing an electrochemical sensor includes transporting a substrate web past at least one print station (as set forth in step 2310) and printing at least one electrochemical sensor electrode on the substrate web at the print station(s). The printing is accomplished by applying an ink composition according to the present invention as described above to the substrate, as set forth in step 2320. As illustrated at step 2330, process 2300 also includes a step of drying the ink composition that has been applied to the substrate at temperature of approximately 140°C with an airflow of 60 m³/min. In one embodiment of the invention, substrate web speed may be 10 m/min

Once apprised of the present disclosure, one skilled in the art will recognize that processes according to the present invention, including process 2300, can be accomplished using methods described in Provisional Patent Application No. 60/436,683.

In one embodiment of the present invention, prior to the carbon printing process and immediately after drying, substrate 242 is passed over a first chilled roller 212 which is designed to rapidly cool substrate 242 to a predetermined temperature, typically room temperature (around 18-21 °C and typically 19.5 °C +/- 0.5°C). In one embodiment of the web manufacturing process according to the present invention the surface of first chilled roller 212 is approximately 18°C. First chilled roller 212 may be cooled to an appropriate temperature using, for example, factory chilled water at around 7°C. The temperature of the roller can be controlled by controlling the flow rate and/or the temperature of the factory chilled water. After the printed carbon patterns are deposited in the printing process, substrate 242 is passed over second chilled roller 218. Reducing the temperature of substrate 242 and maintaining the temperature of substrate 242 is beneficial because cooler temperatures reduces the probability of ink drying on the screens during printing and creating blocks in the mesh. The use of chilled rollers in a web manufacturing process according to the present invention is also beneficial because it reduces the amount of stretch in substrate 242, reducing registration problems and the need to modify the process on the fly to compensate for such problems.

In one embodiment, the temperature of the chilled rollers is controlled dynamically by a feedback loop measuring the temperature of the chilled roller and controlling the water flow/temperature. Other methods of chilling the rollers can be envisaged by those skilled in the art from the embodiments described herein, for example, electrically powered refrigeration units.

In a process according to the present invention, section 4 of the web manufacturing process is where insulation printing takes place. In section 4, the insulation artwork for the electrochemical sensors manufactured in accordance with the present invention is printed utilizing screen-printing utilizing a generally flat screen. The basic components of the insulation print station 104 are illustrated in FIGs. 6 and 7. In particular, a suitable print station according to the present invention includes a screen 301, lower print roller 303, print roller 600, a flood blade 603, a squeegee holder 605 and a squeegee 606. In insulation print station 104, print roller 600 is third print roller 221.

In a flood cycle process in accordance with the present invention, screen 301 is charged with ink 604 by moving squeegee 606, flood blade 603, print roller 600, and lower print roller 303, in first direction 608 which corresponds to the web movement of substrate 242. Screen 301 is moved in second direction 607 opposite to first direction 608 of substrate 242 for the flood cycle where ink 604 is charged onto screen 301.

In a subsequent print cycle process in accordance with the present invention, as illustrated in FIG. 7, squeegee 606 transfers ink 604 through the screen 301 and onto substrate 242. During the print cycle, the squeegee 606, flood blade 603, print roller 600, and lower print roller 303 all move in second direction 607 which is opposite to the web movement of substrate 242. Screen 301 is moved in first direction 608 which corresponds to the web movement of substrate 242 for the print cycle where ink 604 is pushed through screen 301 and deposited on substrate 242. One embodiment of the screen printing mechanism is described in more detail in issued U.S. Patent No. 4,245,554

In movable flat screen printing, during printing a generally flat screen has a component of its motion which is in the same direction and at approximately the same speed as the substrate. Typically in each of the print stations, the substantially flat screen is at an acute angle (A in FIG. 6) to the substrate as the screen and substrate move away from a printing position (adjacent a print roller 200 in FIG. 6). Varying the relative speed of the substrate and the screen varies the size of the printed image in the direction of travel of the substrate, i.e. the X-direction.

The stencil screen used in each of the print stations typically consists of a resiliently deformable polyester or steel mesh stretched and attached to a rigid frame. One embodiment uses a polyester screen supplied by DEK Machinery, Weymouth, UK. The mesh is coated with a UV sensitive coating and in conjunction with a film positive the screen is exposed to a UV light source, developed and dried so that the coating dries on the screen to form a negative of the desired artwork image. With the aid of a squeegee, ink is passed through the open areas of the stencil and onto the substrate (giving a positive image formed by the ink on the substrate). The frame provides a means of mounting the mesh, and withstanding the forces imposed by the stretched mesh with minimum distortion and with standing the additional forces produced during printing.

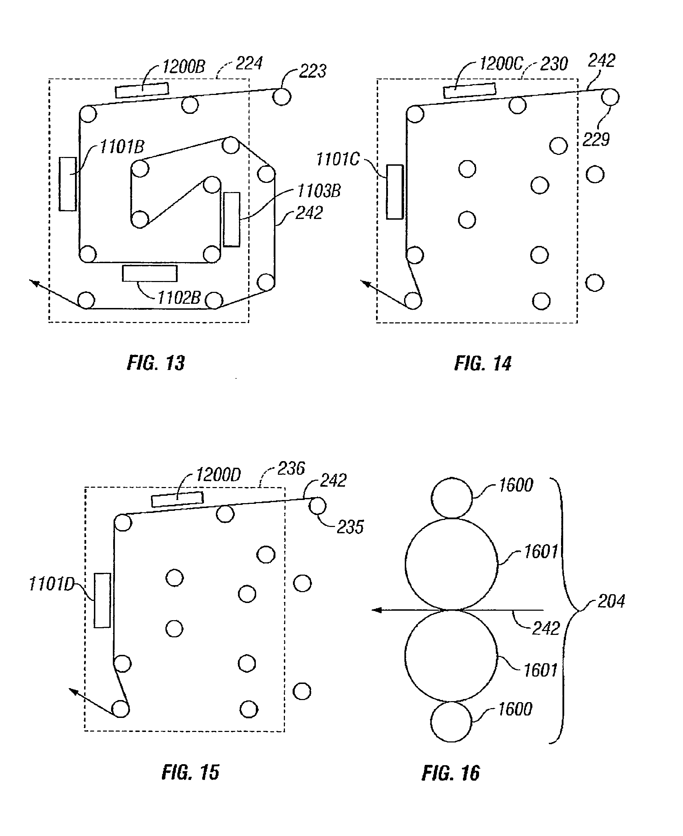

In particular, in insulation print station 104, the ink in question is an insulation ink. An example of a suitable insulation ink is set forth herein below. In this embodiment of the current invention, screen 301 is flooded with ink 604 prior to using squeegee 606 to transfer ink 604 through the screen and onto substrate 242. The printed insulation artwork deposited on substrate 242 is then dried using, for example, hot air at 140 °C directed onto the printed surface of the substrate using four separate drying banks within second drier zone 224, which is illustrated in more detail in FIG. 13. An example of a suitable ink for use in insulation print station in a web manufacturing process according to the present invention is Ercon E6110-116 Jet Black Insulayer Ink which may be purchased from Ercon, Inc. In one embodiment of the invention, insulation artwork is registered to the carbon artwork in the X direction (along the machine) and the Y direction (across the machine) utilizing the techniques described herein. Other types of insulation ink may be utilized as will be understood by those skilled in the art from the description herein. Furthermore different layers or different orders of layers can be used to provide a different order of layers and therefore different construction in the electrochemical sensors produced.

In one embodiment of the present invention, before the insulation printing process and immediately after drying, substrate 242, including printed carbon and insulation patterns, is passed over third chilled roller 219 which is designed to rapidly cool substrate 242 to a predetermined temperature typically room temperature (around 17-21 °C and typically 19.5 °C +/- 0.5 °C). In one embodiment of the web manufacturing process according to the present invention, the surface temperature of the third chilled roller is approximately 18 °C. Third chilled roller 219 may be cooled to an appropriate temperature using, for example, factory chilled water at around 7 °C. Reducing the temperature of substrate 242 and maintaining the temperature of substrate 242 is beneficial because cooler temperatures reduces the probability of ink drying on the screens and creating blocks in the mesh. The use of chilled rollers in a web manufacturing process according to the present invention is also beneficial because it reduces the amount of stretch in substrate 242, reducing registration problems and the need to modify the process on the fly to compensate for such problems.

In a process according to the present invention, section 5 of the web manufacturing process is where the first enzyme printing takes place. In section 5, the enzyme ink artwork for the electrochemical sensors manufactured in accordance with the present invention is printed utilizing screen-printing and a movable generally flat screen as herein before described. The basic components of the first enzyme print station 105 are illustrated in FIGs. 6 and 7. In particular, a suitable print station according to the present invention includes a screen 301, lower print roller 303, print roller 600, a flood blade 603, a squeegee holder 605 and a squeegee 606. In first enzyme print station 105, print roller 600 is fourth print roller 227.

In a flood cycle process in accordance with the present invention, screen 301 is charged with ink 604 by moving squeegee 606, flood blade 603, print roller 600, and lower print roller 303, in first direction 608 which corresponds to the web movement of substrate 242. Screen 301 is moved in second direction 607 opposite to first direction 608 of substrate 242 for the flood cycle where ink 604 is charged onto screen 301.

In a subsequent print cycle process in accordance with the present invention, as illustrated in FIG. 7, squeegee 606 transfers ink 604 through the screen 301 and onto substrate 242. During the print cycle, the squeegee 606, flood blade 603, print roller 600, and lower print roller 303 all move in second direction 607 which is opposite to the web movement of substrate 242. Screen 301 is moved in first direction 608 which corresponds to the web movement of substrate 242 for the print cycle where ink 604 is pushed through screen 301 and deposited on substrate 242. One embodiment of the screen printing mechanism is described in more detail in issued U.S. Patent No. 4,245,554.

In particular, in first enzyme print station 105, the ink in question is an enzyme ink. An example of a suitable enzyme ink is set forth herein below. In this embodiment of the current invention, screen 301 is flooded with ink 604 prior to using squeegee 606 to transfer the ink 604 through the screen and onto substrate 242. The printed enzyme artwork deposited on substrate 242 is then dried using, for example, hot air at 50 °C directed onto the printed surface of the substrate using two separate drying banks within the third drier zone230, which is illustrated in more detail in FIG. 14. An example of a suitable ink for use in first enzyme print station 105 in a web manufacturing process according to the present invention as summarized in Table 2.

**Table 2.**

| Component | Supplier |
|---|---|
| Glucose Oxidase | Biozyme Laboratories |
| Tri-sodium Citrate | Fisher Scientific |
| Citric Acid | Fisher Scientific |
| Poly Vinyl Alcohol | Sigma Aldrich |
| Hydroxyethylcellulose (Nat 250 G) | Honeywell and Stein |
| | BDH/Merck LTD |
| | Sigma-Aldrich Chemical Co., UK |
| Potassium hexacyanoferrate III | Norlab Instruments Ltd., UK |
| DC 1500 Antifoam | BDH/Merck Ltd |
| Cabosil | Ellis and Everard Ltd |
| PVPVA | ISP Company Ltd |
| Analar Water | BDH/Merck Ltd |

In one embodiment of the present invention, after the insulation printing process and immediately after drying, the substrate 242, including printed carbon and insulation patterns, is passed over fourth chilled roller 225 which is designed to rapidly cool substrate 242 to a predetermined temperature typically room temperature (around 17-21 °C and typically 19.5 °C +/- 0.5 °C).. In one embodiment of the web manufacturing process according to the present invention the surface of fourth chilled roller 225 is approximately 18°C. Fourth chilled roller 225 may be cooled to an appropriate temperature using, for example, factory chilled water at around 7°C. Reducing the temperature of substrate 242 and maintaining the temperature of substrate 242 is beneficial because cooler temperatures reduces the probability of ink drying on the screens and creating blocks in the mesh. The use of chilled rollers in a web manufacturing process according to the present invention is also beneficial because it reduces the amount of stretch in substrate 242, reducing registration problems and the need to modify the process on the fly to compensate for such problems.

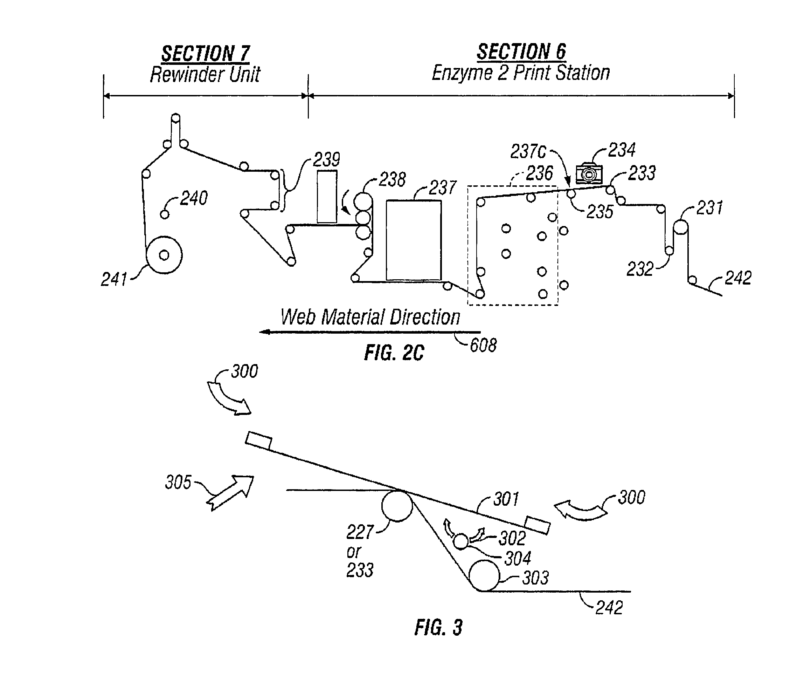

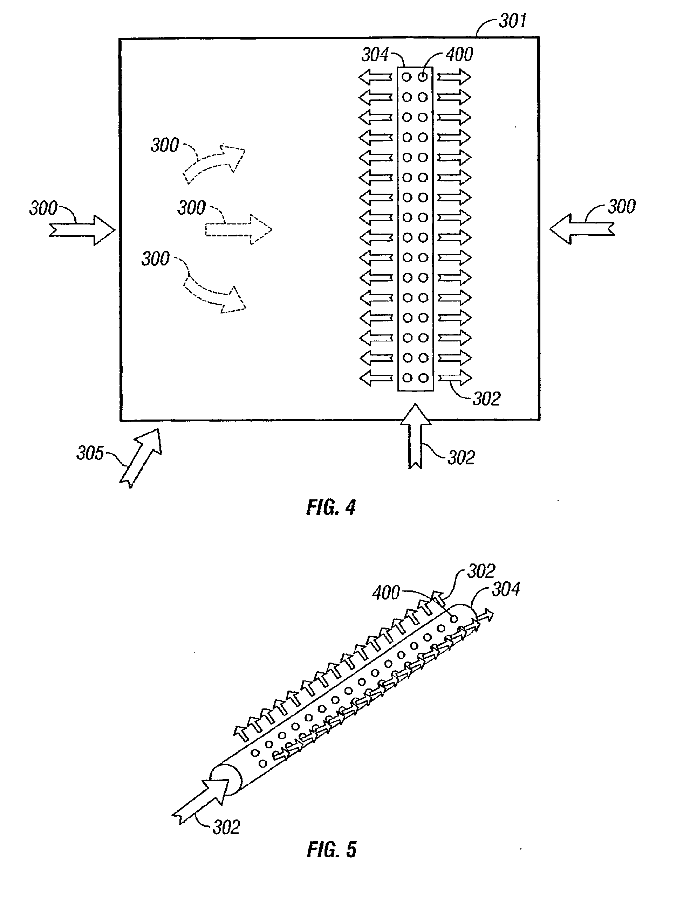

Additionally, due to the high water content of the enzyme ink and the airflow due to the movement of the screen, it is crucial to ensure that the enzyme ink does not dry into the screen. The relative flow of air encountered by the moving screen dries the ink on the screen in a manner not normally observed in flat bed screen printers (such as Thieme flat bed printers) since the screen itself does not move within the machine, unlike the present invention. As well as the chilled roller alleviating this by ensuring the substrate is cooled to around 18 °C before it encounters the enzyme screen-printing step, the screen loaded with enzyme ink is humidified during printing. In one embodiment, humidification is substantially continuous. There may be topside, underside and/or side screen humidification and indeed all three may be provided. An arrangement of pipes provides a substantially constant stream of humidified air above, below and sideways onto the screen respectively, ensuring the water content of the ink, is maintained at a constant level. A suitable arrangement for providing topside, underside and/or side screen humidification according to the present invention is illustrated in FIGs. 3, 4 and 5. The amount and arrangement of humidification means (typically pipes carrying humidified air) will depend, amongst other things, upon the amount of humidification required, the water content of the ink, the humidity and temperature of the surrounding air, the temperature of the substrate as it approaches the enzyme print station, the temperature of the print roller, the size of the screen and the exposure of the screen to the surrounding (unhumidified air). In one embodiment a pipe 304 comprising one or more rows of holes 400 delivers humidified air across the whole underside of the screen during one stroke of the screen back and forth. Pipes (not shown) above and to the operator side of the machine deliver humidified air flows 300 and 304 (see FIG. 4).

Typically all the enzyme ink required for that print run is placed on the screen at or prior to the start of the print run. Since the enzyme ink is composed of a large part of water (typically between 55 and 65% by weight, more typically around 60 % by weight, the ink is prone to drying out over the lifetime of the run. This risk can be alleviated by providing humidification around the screen loaded with enzyme ink. Alternatively or more typically in addition the substrate can be chilled prior to encountering the enzyme (or indeed any) print station by the use of chilled rollers as herein described. Typically the temperature of the substrate is controlled to be less than or equal to the temperature of the room. However, the temperature of the substrate is kept above the dew point for the atmosphere in the room. If the room is at 60% humidity then the dew point may be 15 °C. If the temperature of the substrate falls below this then, condensation can occur on the substrate potentially compromising any subsequent print run, especially any subsequent print run with water soluble ink such as enzyme ink. Control of the substrate temperature, for example between the limits of room temperature and dew point, may therefore be important for a successful print run. Control of temperature of and/or time passing over chilled rollers 212, 219, 225 and 231 is important in controlling substrate temperature. A feedback control loop can be used to measure the substrate temperature for example relative to the room temperature and/or dew point (given the room's humidity) to control the temperature of the chilled rollers and the temperature of the substrate as it leaves the roller and approaches the next print station.

FIG. 2C is a schematic diagram depicting section 6 and section 7 of a web printing process according to the present invention. In FIG. 2C, Section 6 is second enzyme print station 106. Second enzyme print station 106 includes fifth chilled roller 231, fifth accumulator 232, fifth print roller 233, fourth vision sensor 234, fifth drive roller 235, fifth drier zone 236, Y registration system 237 and outbound nip roller 238. In the embodiment of the invention illustrated in FIG. 2C, section 7 is rewinder unit 107. Rewinder unit 107 includes steering mechanism 239, first rewind arbor 240 and second rewind arbor 241.

In a process according to the present invention, section 6 of the web manufacturing process is where the second enzyme printing takes place. In section 6, the enzyme ink artwork for the electrochemical sensors manufactured in accordance with the present invention is printed utilizing screen-printing. The purpose of applying 2 coatings of the enzyme ink is to ensure complete coverage of the carbon electrodes and so that the electrodes are substantially even and free of voids. The basic components of the second enzyme print station 106 are illustrated in FIGs. 6 and 7. In particular, a suitable print station according to the present invention includes a screen 301, lower print roller 303, print roller 600, a flood blade 603, a squeegee holder 605 and a squeegee 606. In second enzyme print station 106, print roller 600 is fifth print roller 233.

In a flood cycle process in accordance with the present invention, screen 301 is charged with ink 604 by moving squeegee 606, flood blade 603, print roller 600, and lower print roller 303, in first direction 608 which corresponds to the web movement of substrate 242. Screen 301 is moved in second direction 607 opposite to first direction 608 of substrate 242 for the flood cycle where ink 604 is charged onto screen 301.

In a subsequent print cycle process in accordance with the present invention, as illustrated in FIG. 7, squeegee 606 transfers ink 604 through the screen 301 and onto substrate 242. During the print cycle, the squeegee 606, flood blade 603, print roller 600, and lower print roller 303 all move in second direction 607 which is opposite to the web movement of substrate 242. Screen 301 is moved in first direction 608 which corresponds to the web movement of substrate 242 for the print cycle where ink 604 is pushed through screen 301 and deposited on substrate 242. One embodiment of the screen printing mechanism is described in more detail in issued U.S. Patent No. 4,245,554.

In particular, in second enzyme print station 106, the ink in question is an enzyme ink. In this embodiment of the current invention, screen 301 is flooded with ink 604 prior to using squeegee 606 to transfer the ink 604 through the screen and onto substrate 242. The printed enzyme artwork deposited on substrate 242 is then dried using, for example, hot air at 50 °C directed onto the printed surface of the substrate using two separate drying banks within a fourth drier zone 236, which is illustrated in more detail in FIG. 15. An example of a suitable ink for use in second enzyme print station 106 is the same as the enzyme ink used in first enzyme print station which is described in aforementioned Table 2.

In one embodiment of the present invention, after the second enzyme printing process and immediately after drying, the substrate 242, including printed carbon, insulation, and enzyme ink patterns, is passed over fifth chilled roller 231 which is designed to rapidly cool substrate 242 to a predetermined temperature. In one embodiment of the web manufacturing process according to the present invention the surface of the fifth chilled roller 231 is approximately 18 °C. Fifth chilled roller 231 may be cooled to an appropriate temperature using, for example, factory chilled water at around 7 °C. Reducing the temperature of substrate 242 and maintaining the temperature of substrate 242 is beneficial because cooler temperatures reduces the probability of ink drying on the screens and creating blocks in the mesh. The use of chilled rollers in a web manufacturing process according to the present invention can also be beneficial because it reduces the amount of stretch in substrate 242, reducing registration problems and the need to modify the process on the fly to compensate for such problems.

Additionally, due to the high water content of the enzyme ink and the airflow due to the movement of the screen, it is crucial to ensure that the enzyme ink does not dry into the screen. As well as the chilled roller alleviating this by ensuring the substrate is cooled to 18 °C before it encounters the enzyme screen-printing step, there is also topside and/or underside and/or side screen humidification, which can provide a stream of humidified air above and below the screen, ensuring the water content of the ink is maintained at a constant level. Typically the humidified air flows constantly over the screen. A suitable arrangement for providing topside and underside screen humidification according to the present invention is illustrated in FIG. 3.

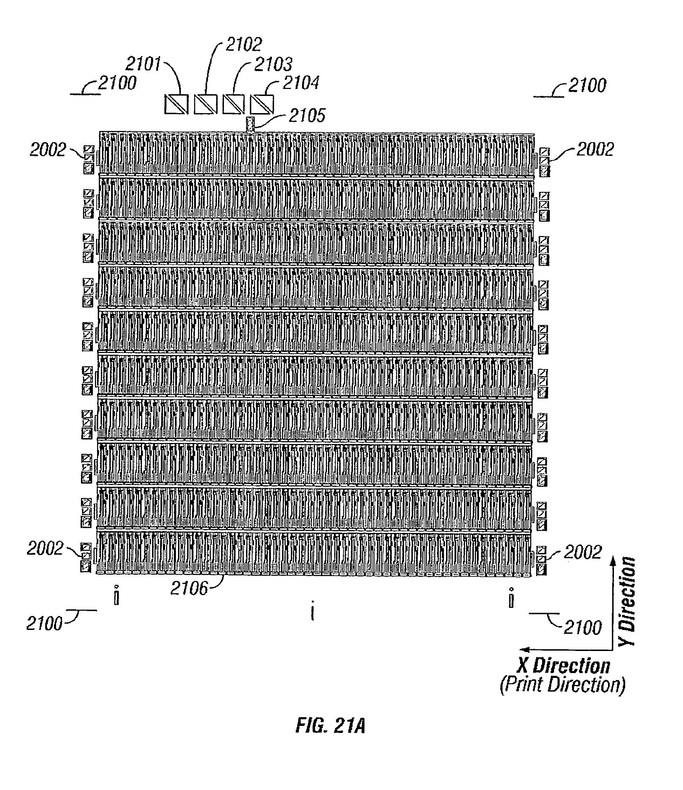

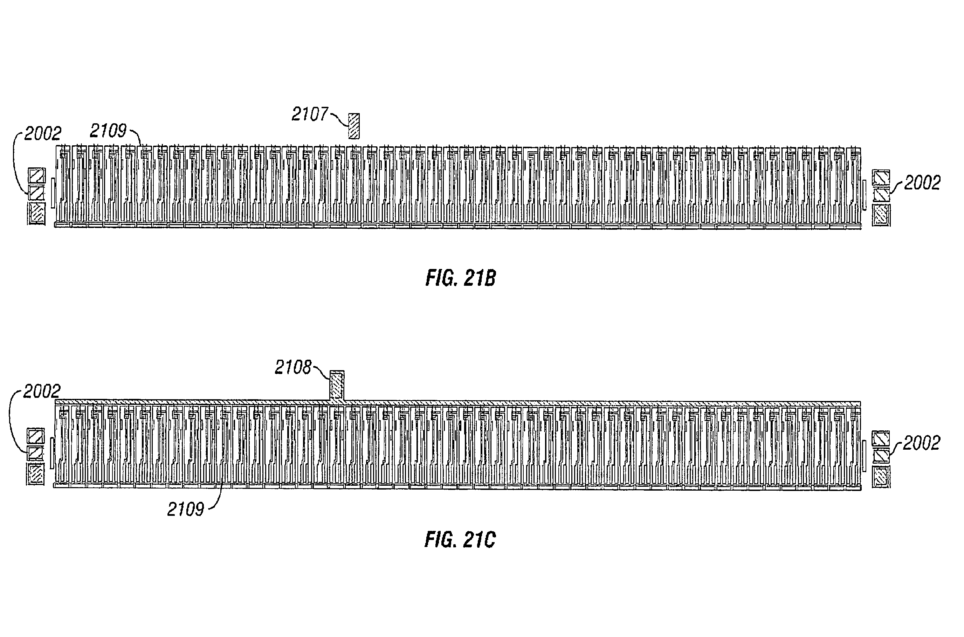

Second enzyme print station 106 may include outbound nip roller 238, inspection system 237 for inspecting registration, third Y registration system at 237C (not shown) and barcode station (not shown). Outbound nip roller 238 helps control the tension of substrate 242 (specifically the tension between inbound nip roller 206 and outbound nip roller 238). Substrate 242 is removed from second enzyme print station 106 at a constant rate by outbound nip roller 238. The Y registration system (not shown) at positons 237A, 237 B and 237C controls the Y registration (i.e. across the web) of each print cycle during printing by utilizing the first Y registration marks 2101, second Y registration marks 2102, third Y registration marks 2103, fourth Y registration marks 2104 which are illustrated in Fig 21A. In one embodiment of the invention, first Y registration marks 2101, second Y registration marks 2102, third Y registration marks 2103, and fourth Y registration marks 2104 may correspond, respectively, to the Y registration of carbon print station 103, insulation print station 104, first enzyme print station 105, and second enzyme print station 106. Each Y registration marks comprises 2 triangles that are juxtaposed in an orientation that approximates a rectangle. In one embodiment the Y registration system located at positions 237A, 237B and 237C can be implemented by an Eltromat DGC650 from Eltromat Gmbh in Leopoldshöhe, Germany.

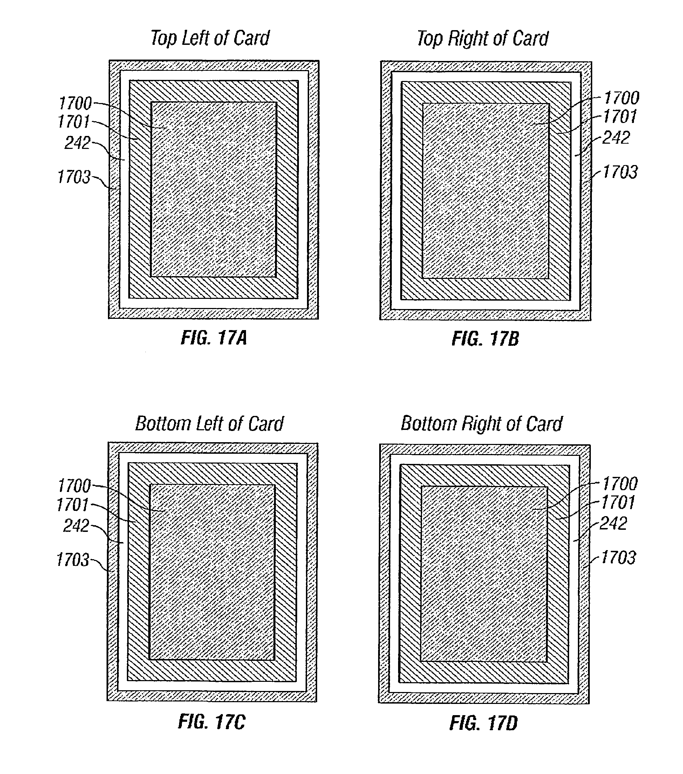

In one embodiment of the present invention, the inspection system 237, may be implemented using the Eltromat Inspection System, model number PC3100 HD, which is commercially available from Eltromat Gmbh in Leopoldshöhe, Germany. The inspection system237 has a vision component that inspects the registration marks illustrated in FIGs. 17A to 19D and/or FIG. 20D and can be used as a tool in assessing whether a sensor sheet 2106 should be rejected (for example by recording inspection results against a barcode in a database).

Registration issues in the Y dimension (which can be altered during printing by the registration system (not shown) which is located at 237A, 237B and 237C and/or inspected by inspection system 237 after all print stages are complete) may be ascribed to variations in web tension or non-uniform distortions to the substrate 242. In an embodiment of the invention, the barcode station comprises the following commercially available components barcode printer (model number A400 from Domino UK Ltd. In Cambridge, United Kingdom), barcode traverse system (Scottish Robotic Systems in Perthshire, Scotland), and barcode reader (RVSI Acuity CiMatrix in Canton, MA). The barcode station (no shown) labels each row of the sensor sheet 2106 with a 2 dimensional bar code. This provides each row of sensors a unique identifier code, batch/Lot number identification, the sensor sheet number, and row number. The barcode station also reads barcode immediately after printing to verify that the barcode has printed properly and provides a visual indicator to the machine operators. The barcode and process information from sections 2 to 6 are stored in a database and used later to identify and subsequently reject/accept cards for future process.

Rewinder unit 107 consists of, for example, a Martin Automatic Rewind System. This is the last section of the machine and allows the continuous rewind of substrate 242. Rewinder unit 107 consists of a first rewind arbor 240 and second rewind arbor 241. First rewind arbor 240 holds a roll of substrate material 242 and continuously pulls material from second enzyme print station 106. Second rewind arbor 241 holds a standby roll of material, which automatically splices a first roll of substrate 242 into a second roll on the completion of the roll of substrate 242 from first rewind arbor 240. This continuous process repeats from first rewind arbor 240 to second rewind arbor 241. A flying splice, which occurs while the substrate 242 is still moving, is used to enable the continuous rewind of substrate 242. The splice is placed directly onto a fresh roll of substrate material 242 which is primed with double sided pressure sensitive adhesive.

FIG. 3 is a schematic diagram depicting the humid environment around a fifth and sixth sections of the web printing. The basic components used to provide the means for humidification of the web printing environment are illustrated in FIG. 3 which includes top humid air 300, screen 301, bottom humid air 302, lower print roller 303, pipe 304 comprising multiple perforations 400, substrate 242, and either fourth print roller 227 or fifth print roller 233. Humidification and temperature is set to try and ensure that the properties of the enzyme ink do not change to any significant extent over time during the flood and print cycle and preferably over the life of the print run. In particular, it is desirable that the viscosity and water content of the enzyme ink not change over time during the flood and print cycle and preferably over the life of the print run.. The enzyme ink is approximately 63% water. A constant water content ensures that the amount of ink laid down onto the substrate 242 is consistent. If the water content of the ink changes during the printing process, this can lead to variations in the enzyme layer thickness. Additionally, loss of moisture from the enzyme ink shall lead to the enzyme drying on screen 301 resulting in poor print definition and a reduction in the amount of ink laid onto substrate 242. The humid air inside either first enzyme print station 105 or second enzyme print station 106 is maintained between 85 to 95% relative humidity. Top humid air 300 and bottom humid air 302 is pumped onto both sides of screen 301 to maintain the desired relative humidity. A side pipe 305 is arranged to one side of the web and introduces humidified air to the web on one side immediately adjacent the enzyme print stations. The nature and type of humidification arrangements can be varied to suit the size and shape of the print station and the humidification requirements of that type of ink at that print station in that environment. Often a hood can be used to enclose the upper and/or lower side of the screen so that humidified air can be delivered into the hood directly adjacent the screen and retained within the vicinity of the screen by the presence of the hood. If the hood is mounted on the upper screen frame, as is typically the case, the hood may have a slot in the x direction (the direction of printing) to allow the squeegee to move in relation to the screen during the normal flood/print cycle.

FIG. 4 is a bottom view depicting the humid environment around a fifth and sixth sections of the web printing. The basic components used to provide the means for humidification of the web printing environment are also illustrated in FIG. 4 which includes top humid air 300, screen 301, bottom humid air 302, pipe with perforations 304, and perforations 400, side pipe at 305 (not shown). A pipe 304 with several perforations 400 is positioned underneath screen 301 as a means for blowing bottom humid air 302 to maintain the viscosity of the enzyme ink on screen 301. FIG. 5 is a perspective view of pipe 304 with perforations 400 to blow bottom humid air 302.

FIG. 8 is a schematic diagram depicting 2 different squeegee angles which includes a substrate 242, print roller 600, and squeegee 606. The angle of the squeegee 800 can be varied to optimize the definition of the print area. In an embodiment of the invention the angle of the squeegee can be 15 +/-5 and preferably +/-1 to 2 degrees. Note that the contact point of the squeegee 606 to print roller 600 is the same for every squeegee angle 800.

FIG. 9 is a schematic diagram depicting 2 different squeegee positions which includes substrate 242, print roller 600, lower print roller 303, squeegee 606, first squeegee position 900, and second squeegee position 901. The squeegee position is the position of the squeegee relative to the center of the print roller 600. The squeegee position can have a major effect on the thickness of printed ink. The position of the squeegee can be varied to optimize the definition of the print area.

FIG. 10 is a schematic diagram depicting a screen snap distance (1000) which includes substrate 242, print roller 600, lower print roller 303, and screen 301. In one embodiment of the invention, screen snap distance (1000) is the closest distance between screen 301 and substrate 242. In a preferred embodiment of this invention, screen snap setting (1000) may be approximately 0.7mm. If the screen snap setting (1000) is set too high, squeegee 606 cannot sufficiently deflect screen 301 to transfer ink 604 onto substrate 242 with sufficient print definition. If the screen snap setting (1000) is set too low, screen 301 will smear ink 604 from a previous print cycle causing insufficient print definition.

FIG. 11 illustrates an exploded view of a preconditioning zone 211 which comprises first drive roller 210, hot plate 1100, first heater bank 1101, second heater bank 1102, and third heater bank 1103. In an embodiment of the invention, hot plate 1100 contacts the unprinted side of substrate 242. In a preferred embodiment of this invention, hot plate 1100 may be coated with Teflon and may be heated to approximately 160 °C. In an embodiment of the invention, first heater bank 1101, second heater bank 1102, and third heater bank 1103 blow hot air at approximately 160 °C. This may be varied to suit the substrate type and/or thickness and/or any pretreatment and/or later temperatures encountered in the process as would be understood by those skilled in the art.

FIG. 12 illustrates an exploded view of a first drying zone 217 which comprises second chilled roller 218, second drive roller 216, first drier bank 1200A, second drier bank 1101A, third drier bank 1102A, and fourth drier bank 1103A. In an embodiment of the invention, first drier bank 1200A, second drier bank 1101A, third drier bank 1102A, and fourth drier bank 1103A blow hot air at approximately 140 °C although this may be varied as would be understood by those skilled in the art from the description herein.

FIG. 13 illustrates an exploded view of a second drying zone 224 which comprises third drive roller 223, first drier bank 1200B, second drier bank 1101B, third drier bank 1102B, and fourth drier bank 1103B. In an embodiment of the invention, first drier bank 1200B, second drier bank 1101B, third drier bank 1102B, and fourth drier bank 1103B blows hot air at approximately 140 °C although this may be varied as would be understood by those skilled in the art from the description herein.

FIG. 14 illustrates an exploded view of a third drying zone 230 which comprises fourth drive roller 229, first drier bank 1200C, and second drier bank 1101C. In an embodiment of the invention, first drier bank 1200C and second drier bank 1101C, blows hot air at approximately 50 °C although this may be varied as would be understood by those skilled in the art from the description herein.

FIG. 15 illustrates an exploded view of a fourth drying zone 236 which comprises fifth drive roller 235, first drier bank 1200D, and second drier bank 1101D. In an embodiment of the invention, first drier bank 1200D and second drier bank 1101D, blows hot air at approximately 50 °C although this may be varied as would be understood by those skilled in the art from the description herein.

FIG. 16 illustrates an exploded view of a first cleaning unit 204 which comprises tacky rollers 1600, blue polymer rollers 1601. In an embodiment of the invention, blue polymer rollers 1601 contact the top and bottom side of substrate 242 and transfers particulate/foreign material to tacky rollers 1600.

FIGs. 17A to 17D illustrate views of an insulation layer to carbon layer print for an embodiment of the invention with proper registration. Note that FIG. 17A represents the top left, FIG. 17B the top right, FIG. 17C the bottom left, and FIG. 17D the bottom right of sensor sheet 2106. The marks are not shown on the sensor sheet illustrated in FIG. 21 A. In one embodiment of this invention, carbon print station 103 prints carbon layer which comprises a solid carbon rectangle 1700 surrounded by a rectangular carbon line 1703 onto substrate 242. In a subsequent print cycle, insulation print station 104 prints rectangular insulation line 1701 onto substrate 242 which is positioned in between the solid carbon rectangle 1700 and the rectangular carbon line 1703. When the insulation layer to carbon layer registration is proper at all four corners typically there may be no uncoated substrate 242 showing between the rectangular insulating line 1701 and solid carbon rectangle 1700. The registration of insulation layer to carbon layer can be checked manually by an operator or can be checked using second vision sensor 222, which in one embodiment comprises a camera pointed at each corner of the substrate. Typically this forms part of the initialization at the start of the print run. An operator can view all four corners of the substrate adjacent one another on a TV screen. The operator can then visually inspect the registration of insulation to carbon during this initialization process (and indeed during the remainder of the print run) and can make whatever adjustments are necessary to bring the insulation and carbon prints into registration. It should be appreciated that the web viewer 222 (comprising, for example, 4 cameras pointed at locations above four corner of the substrate card) views and forwards for display a snapshot of each of the four corners of each card. Thus the corners of each card are only viewed for a fraction of a second on the display since the substrate beneath the viewing cameras is constantly being replaced as the web travels through the apparatus. This system enables an operator to see instantly the effects any adjustment he may make has on the insulation to carbon registration. Adjustments the operator may make include, but are not limited to, screen print stroke, snap height, squeegee pressure, screen position relative to "Y" direction, screen position in relation to θ (Theta). Once the viewer registration has been set up on this and other print stations (using viewers 228 and 234) the automatic internal X registration system (using marks 2107 and 2108) and the automatic Y registration system (for example, registration systems located at positions 237A, 237B and 237C using marks 2101 to 2104) are allowed to take over and monitor and automatically correct X and Y registration during printing. Marks 1700 to 1703 shown in FIGs. 17A to 20 D can be used for automatic X and Y registration during printing as an alternative or in addition to using marks 2101 to 2104 and 2107 and 2108 as would be understood by those skilled in the art from the description herein.

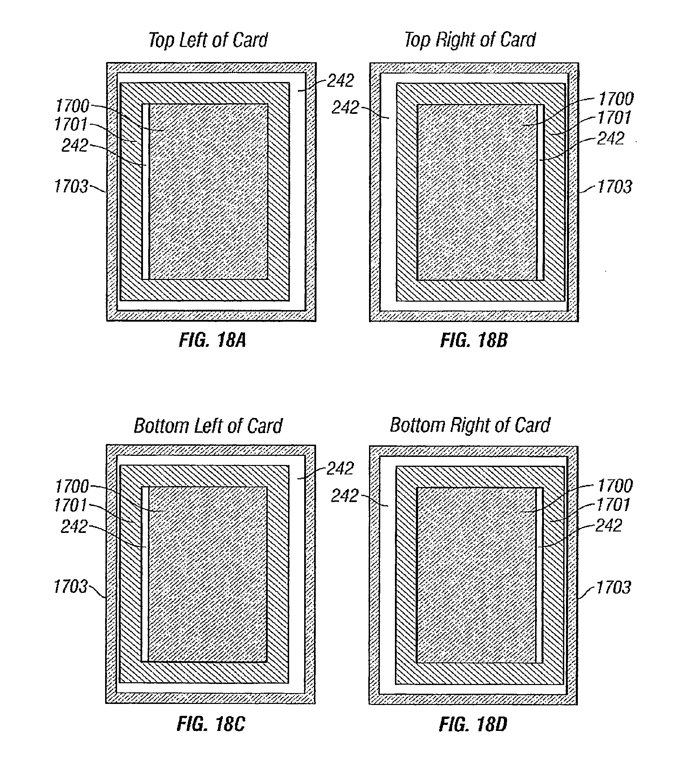

FIG. 18 illustrates a view of an insulation layer to carbon layer for an embodiment of the invention with improper registration when the insulation artwork is longer in the direction of printing than the carbon artwork. This may occur even if the carbon and insulation screen are the same size in this dimension because of the substrate may have stretched or the screen stroke may be different in each stage (a slower screen stroke gives a relatively longer artwork print along the direction of travel of the substrate web). Note that FIG. 18A represents the top left, FIG. 18B the top right, FIG. 18C the bottom left, and FIG. 18D the bottom right of sensor sheet 2106. When the insulation layer to carbon layer registration is improper at one of the four corners uncoated substrate 242 can be observed between the rectangular insulating line 1701 and solid carbon rectangle 1700. The registration of insulation layer to carbon layer can be checked manually by an operator using second vision sensor 222.

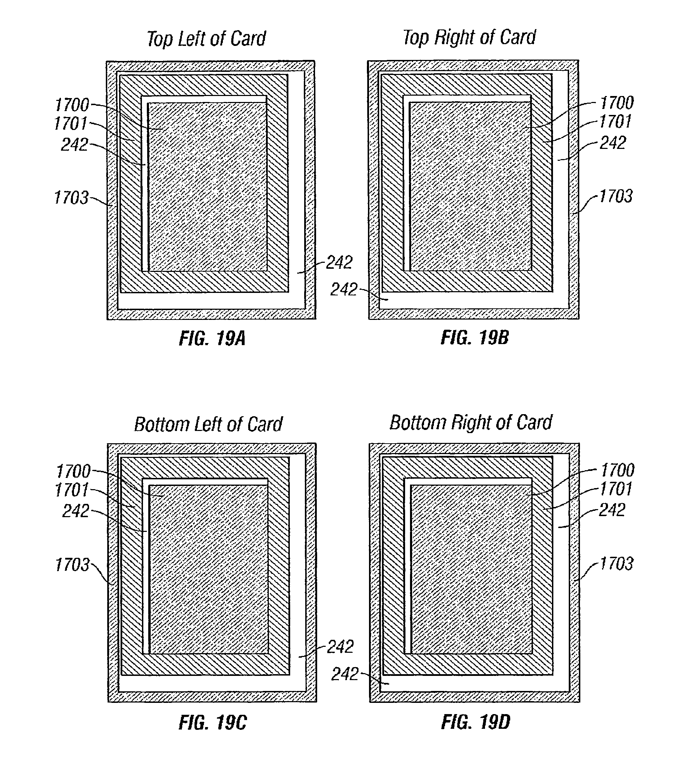

FIG. 19 illustrates a view of an insulation layer to carbon layer for an embodiment of the invention with improper registration when the printed insulation artwork is shorter than that of the carbon print (for example, the screen stroke for the insulation print may be longer than that of the carbon, or the insulation screen may be shorter than that of the carbon print station). Note that FIG. 19A represents the top left, FIG. 19B the top right, FIG. 19C the bottom left, and FIG. 19D the bottom right of sensor sheet 2106. When the insulation layer to carbon layer registration is improper at one of the four corners uncoated substrate 242 can be observed between the rectangular insulating line 1701 and solid carbon rectangle 1700. The registration of insulation layer to carbon layer can be checked manually by an operator using second vision sensor 222.

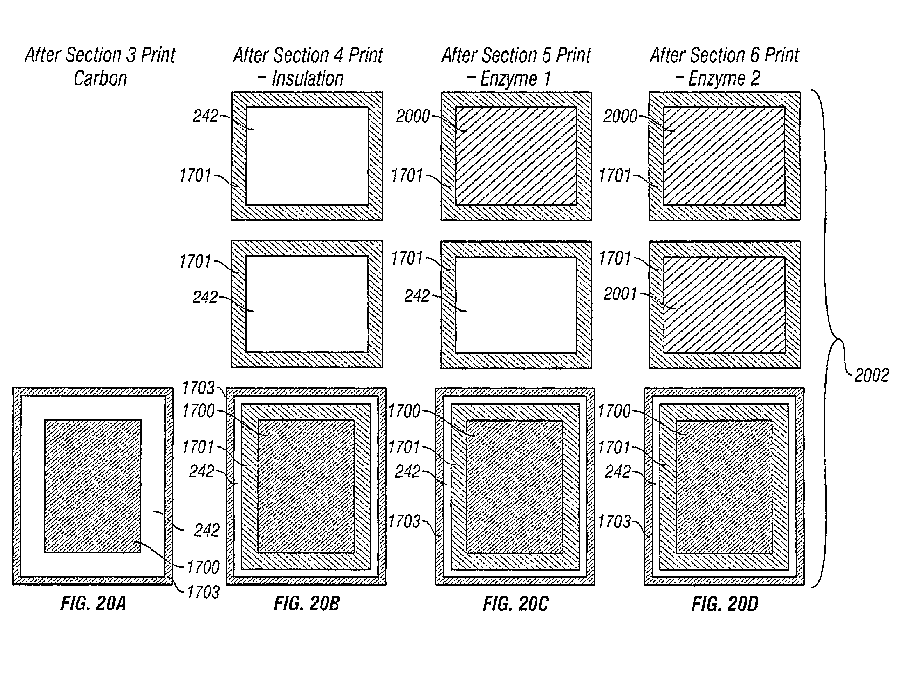

FIGs. 20A to 20D are schematic diagrams depicting the results of a process for printing a second view guide 2002 (see FIG. 21A) which comprises solid carbon rectangle 1700, hollow insulation rectangular line 1701, hollow carbon rectangle 1703, solid rectangle from the first enzyme layer 2000, solid rectangle from the second enzyme layer 2001, and uncoated substrate 242. Optionally, such prints can also be used during manufacture by automatic ongoing inspection systems such as inspection system 237 in section 6 (after the second enzyme print). Ongoing registration is typically otherwise carried out by a registration system (not shown) at positions 237A, 237B and 237C in the "Y" direction and by a registration control system looking at marks 2105 (see FIG. 21 A) in the "X" direction).

FIG. 21A is an example of a sensor sheet with a first view guide 2100 and second view guide 2002; first Y registration marks 2101, second Y registration marks 2102, third Y registration marks 2103, and fourth Y registration marks 2104; and X registration marks 2105. Note that X registration marks 2105 comprises carbon X registration mark 2107 and insulation X registration mark 2108. FIG. 21B is an exploded view of one row within sensor sheet 2106 with a carbon X registration mark 2107 and second view guide 2002. FIG. 21C is an exploded view of one row within sensor sheet 2106 with an insulation X registration mark 2108 and second view guide 2002. Insulation X mark 2108 entirely overcoats carbon X registration mark 2107 as illustrated in FIG. 21C and in doing so provides a trigger point (left hand edge say of mark 2108) in advance of that of the original carbon mark 2107. This means that any subsequent layers are printed in relation to the second printed layer (in this case the insulation layer) rather than the carbon layer. This can be useful say if the second and subsequent screen artwork dimensions are longer in the X direction (along the web) than the first screen artwork dimension in the X direction.

An exploded view of one corner of the print guides is shown in FIGS. 20A-D, in the sequence in which they are printed. At section 3 of carbon print station 103, a solid carbon rectangle 1700 is printed along with a rectangular carbon line 1703, which surrounds the solid carbon rectangle 1700. At section 4 of insulation print station 104, a rectangular insulation line 1701 is printed between the solid carbon rectangle 1700 and the rectangular carbon line 1703. When insulation to carbon registration is correct at all four corners typically there will be no uncoated substrate 242 showing between solid carbon rectangle 1700 and rectangular insulating line 1701. Additionally, at section 4 of insulation print station 104, there are two more rectangular insulation lines 1701 printed directly above the solid carbon rectangle 1700. These two additional insulation lines are used to visually assess the registration of first enzyme layer 2000 to the insulation layer and second enzyme layer 2001 to the insulation layer, this is done so by printing a solid rectangle of enzyme ink within the rectangular insulation line as illustrated in FIGs. 20C and 20D. Thus the third and fourth printed layers can be registered to the second and not to the first printed layers. This has the advantage that a change in artwork size between the first and second layers (which may be required should the substrate stretch after the first print station for example due to the heat and tension encountered in the first drying zone 217) can be accommodated without adverse effect on print registration (a tolerance of 300 µm is typical in the X direction).

As illustrated in FIGs. 1 and 2, at the end of the process, substrate 242, including the sensors printed thereon is rewound by rewinder unit 107 and is then fed into punch 108, which may be, for example, a Preco punch which is located within a low humidity environment. The Preco Punch is a CCD X, Y, Theta, Floating Bolster Punch. The Preco Punch registration system uses a CCD vision system to look at "Preco Dots" which are printed on the Carbon print station, these allow the punch to adjust to the carbon print and enable the punch to "punch" the cards out square. The output of Punch 108 is a set of punched cards such as those illustrated in FIG. 21A. Punched cards are ejected from punch 108 onto a conveyer belt, this conveyer belt transports the cards under a barcode reader which reads two of the barcodes on each card to identify whether the card is accept or reject in relation to the Web Database. Automatic or manual extraction of rejected cards can carried out. The cards are then stacked on top of one another in preparation for the next manufacturing step.

At carbon print station 103, insulation print station 104, first enzyme print station 105, and second enzyme print station 106 all have a means for visually inspecting the registration immediately after the printing process step using first vision sensor 215, second vision sensor 222, third vision sensor 228, fourth vision sensor 234, respectively. For each section in the web printing manufacturing process - Section 3, 4, 5 and 6 - there are Web Viewer camera systems located immediately after the printing process step (See FIGs. 2A-2C for web viewer locations). There are two cameras at Section 3 and four cameras each at Section 4, 5 and 6. The web viewer cameras are part of a manual set-up process used by the Web machine operators during the start of the print run. The cameras are used to view printed marks, which aid the initial set-up of carbon alignment to substrate 242 and registration between insulation layer to carbon layer, first enzyme layer to insulation layer, and second enzyme layer to insulation layer. The printing guides are illustrated indicated on FIG. 21A. For carbon print alignment, second view guide 2100 is used to indicate the carbon print position in relation to the edge of substrate 242 as it runs through carbon print station 103. There is a leading line and a trailing line as illustrated in FIG. 21 A. The carbon print is adjusted until the lines indicate that the print is square to the substrate edge. Registration of the individually printed layers is required in the X direction (along the length of the machine) and the Y direction (across the width of the machine) See FIG. 21 A. X direction registration is controlled by the internal registration system of the machine. This utilizes the printed areas indicated on FIGs. 21A, B and C. On the Carbon print cycle a carbon X registration mark 2107 is printed in this area. The Insulation printing cycle is registered to the Carbon print using sensors which use carbon X registration mark 2107 to allow the insulation screen to adjust in order to print the insulation ink in the correct position. The carbon X registration mark 2107 used for this purpose is then over printed with insulation X registration mark 2108 and is utilized in the same manner to correctly register first enzyme layer 2000 and second enzyme layer 2001 with the insulation print. Y direction registration is controlled by Y registration system (not shown) located at positions 237A, 237B and 237G which in one embodiment of the invention may be an Eltromat registration system, model number DGC650 from Leopoldshöhe, Germany. This utilizes the printed areas 2101 to 2104 indicated in FIG. 21 A. On each print cycle - Carbon, Insulation, Enzymel and Enzyme2 - these marks are printed in order that the subsequent print is registered, via sensors, in the Y direction. The Web Database records process information during printing. Information recorded in the database can be traced back to each individual card via a barcode, in one embodiment a 2D barcode is used. Typical information gathered in the Web database is outlined in Table 3. The Web Database has the ability to assess whether a process parameter is Acceptable or Unacceptable and can be used to reject cards on this basis - whether the parameters were running within there tolerance limit. Unacceptable cards may be removed at future processes either manually or automatically.

**Table 3.**

| **Section 2 -** | **Section 3 -** | **Section 4 -** | **Section 5 -** | **[00118] Section 6 -** |
|---|---|---|---|---|
| **Pre Condition** | **Carbon** | **Insulation** | **Enzyme 1** | **Enzyme 2** |
| **Hot Plate 1 of 1** | **Drier Bank 1 of 2** | **Drier Bank 1 of 3** | **Drier Bank 1 of 4** | **Drier Bank 1 of 4** |
| **Drier Bank 2 of 1** | **Drier Bank 2 of 2** | **Drier Bank 2 of 3** | **Drier Bank 2 of 4** | **Drier Bank 2 of 4** |
| **Drier Bank 3 of 1** | **Drier Bank 3 of 2** | **Drier Bank 3 of 3** | **Squeegee Pressure** | **Squeegee Pressure** |
| **Drier Bank 4 of 1** | **Drier Bank 4 of 2** | **Drier Bank 4 of 3** | **Inside Hood %RH** | **Inside Hood %RH** |
| | **Squeegee Pressure** | **Squeegee Pressure** | **Inside Hood Temp** | **Inside Hood Temp** |
| | | | **Outside Hood %RH** | **Outside Hood %RH** |
| | | | **Outside Hood Temp** | **Outside Hood Temp** |
| **Other** | | | | |
| **Web Tension** | | | | |
| **Web Speed** | | | | |

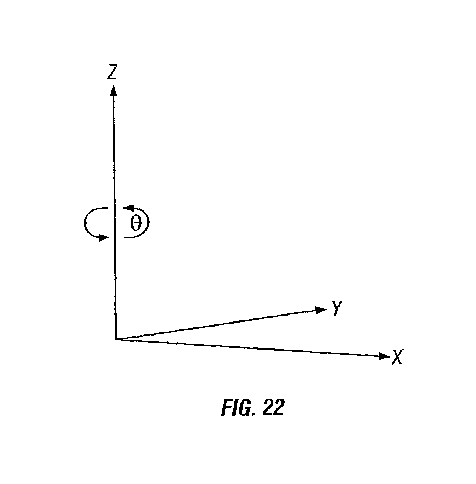

FIG. 22 is a schematic diagram of parameters X, Y, Z and θ used to register the web printing process. The parameter Y represents the direction from the operator to the machine side of the web printing machine (typically horizontal). The parameter X represents the direction from unwind unit 101 to rewinder unit 107 (typically horizontal). The parameter Z represents the direction perpendicular to the X and Y directions (typically vertical). The parameter θ represents the angle around the Z axis. In an embodiment of this invention, the following parameters are used to register the following print process such as, for example, carbon print station 103, insulation print station 104, first enzyme print station 105, and second enzyme print station 106.

In one embodiment of the present invention, the output of the web manufacturing process is cards printed with artwork comprising Carbon, Insulation and two identical Enzyme layers printed in register with one another to form strips each containing an electrochemical sensor and associated contact electrodes for detecting Glucose in a blood sample. The strips are used for self-monitoring of blood glucose in conjunction with a meter. Productions of several designs of strips are envisaged. At present the web is designed to produce "One Touch Ultra" strips for use in the One Touch Ultra meter which is available from LifeScan, Inc.

A schematic diagram sample of the artwork produced is in FIG. 21A. This illustrates one complete printed card, which contains 10 "Rows" of 50 "Strips". There are a total of 500 "Strips" per card. Print orientations are also indicated. By printing rows 0 to 9 (each of 50 strips) parallel to the direction of print, the process can be easily extended to inclusion of a cutting step separating one row from another. Furthermore this means that any defective rows resulting from cross web variation in print quality (perpendicular to the direction of print)can be identified easily. Each row is allocated a number (identified by a barcode) and therefore specific rows from specific sheets on the web can later be identified with reference to the database and eliminated without the need to reject the whole sheet. This increases the yield of usable product from the process and renders the whole process more efficient.

The movable substantially flat screen copes well with the types of ink (solid/liquid combinations) used in the printing of electrochemical sensors. The use of a movable flat screen can enable better control of print definition and the deposition of the thicker layers of ink needed in electrochemical sensors than may be allowed by rotogravure or cylinder screen printing. A variety of types of screen (with different mesh, diameter of thread in the mesh, thread separation, thickness, mesh count) are readily commercially available to cope with the different requirements of different types of ink in the continuous web printing process (carbon, insulation, enzyme).

Because of the arrangement of the flat screen, print roller, substrate and a squeegee urging the screen towards the substrate, a variety of parameters are available to be manipulated (screen to substrate angle, squeegee angle, screen to squeegee position, squeegee to print roller position, snap distance, relative speeds of substrate and screen and squeegee etc) to optimize the print process for electrochemical sensors.

To summarize briefly in a web manufacturing process for manufacturing electrochemical sensors, the web expands or stretches as it is heated up and placed under tension during the process. The printing stations (for example carbon, insulation, two enzyme) typically each are followed by a drying station. In order to dry the inks efficiently the drier stations operate at quite high temperatures (50-140 degrees centigrade). Furthermore to aid registration of the web through each printing station, the web is placed under tension.

The substrate has to be kept under tension to control registration within the process, as a result, whenever the substrate is heated for example to dry the inks after printing, the substrate will stretch unpredictably causing image size variation in subsequent prints.

The size of the image printed at each print station is determined by several factors (stencil size, ink viscosity, relative web and stencil/screen speed and substrate stretch at that point (both reversible and irreversible stretch) etcThe image size variation (between different printing steps) when looked at the end of the process was found to vary. It was unpredictable and higher than expected significantly reducing yields. If the mismatch between image sizes between layers is greater than 300microns along the web (x-direction), the product will not work. The excessive image size variation was thought to be due to excessive and unpredictable stretching (due to heating and tension) and shrinking of the web substrate.

The problem of stretch and tension does not cause the same problems in flat bed printing. To solve the problem in the web process, pre-shrunk substrate was tried. The substrate was heated to around 185 degrees centigrade before being used in the web process. However, the variation in image size remained a problem, and caused reduced yields.

The current proposal for the web process is the use of high temperatures in a first drier or rather preconditioned at a sufficiently high temperature so that in one example, irreversible stretch is substantially removed from the substrate, prior to an image being printed on the substrate.

In a first processing station in the web machine, a drier bank heats the substrate up to 160 degrees centigrade. The temperatures encountered by the substrate later in the process, typically do not exceed 140 degrees.

In FIG. 2A the first heater bank that the unprinted substrate encounters is the hot plate. This is a Teflon coated plate, which lifts and contacts the substrate during motion of the web. The heat is introduced to the back face of the substrate. This is currently running at a set point of 160 °C with a specification of +/- 4 °C. The 160 °C set point statistically provided the best dimensional control. The calculated mean is 160.9 °C. In Bank 2 hot air is introduced to the front face of the substrate at a set point of 160 °C with a specification of +/- 4°C. The calculated mean is 161.29 °C. In Bank 3 hot air is introduced to the front face of the substrate at a set point of 160 °C with a specification of +/- 4 °C. The calculated mean is 161.18 °C. In Bank 4 hot air is introduced to the front face of the substrate at a set point of 160 °C with a specification of +/- 4 °C. The calculated mean is 160.70 °C.

As a result of the web tension and the heat introduced in the drier, the web substrate is stretched by approximately 0.7 mm per artwork repeat. This was one of the primary reasons for utilizing Station 1 as a preconditioning unit to stabilize the substrate prior to subsequent printing stations. The use of Station 1 to precondition the substrate improves the stability of Carbon and Insulation Row Length since much of the material stretch has been removed from the substrate prior to printing.

It will be recognized that equivalent structures may be substituted for the structures illustrated and described herein and that the described embodiment of the invention is not the only structure which may be employed to implement the claimed invention. In addition, it should be understood that every structure described above has a function and such structure can be referred to as a means for performing that function.

## Claims

1. A method of manufacturing an electrochemical sensor comprising a substrate, and at least two layers to be printed on the substrate, the method comprising transporting a web of the substrate past first and second print stations, a drying station, positioned between said print stations, and a cooling station, located between the drying station and the second print station; printing a conductive ink layer on the substrate as it is transported past the first print station by applying a conductive ink composition to the substrate, the conductive ink composition comprises:
graphite;
carbon black;
a resin; and
at least one solvent;
wherein a weight ratio of graphite to carbon black is in a range of from 4:1 to 1:4; and
wherein a weight ratio of a sum of graphite and carbon black to resin is in a range of from 10:1 1 to 1:1;
drying the first conductive ink layer on the substrate in the drying station and cooling the printed substrate in the cooling station before printing a second layer on the substrate as it is transported past a second print station.

2. The method of claim 1, wherein the solvent in the conductive ink composition has a boiling point between 120 °C and 250 °C.

3. The method of claim 1, wherein the solvent in the conductive ink composition includes isophorone, diacetone alcohol and methoxy propoxy propanol.

4. The method of claim 1, wherein the resin in the conductive ink composition is a terpolymer that includes vinyl chloride, vinyl acetate and vinyl alcohol.

5. The method of claim 1, wherein the ratio of graphite to carbon black in the conductive ink composition is 2.62:1 and the ratio of the sum of graphite and carbon black to resin is 2.9:1.

6. The method of claim 1, wherein a particle size of the graphite in the conductive ink composition is 15 µm.

7. The method of claim 1, wherein the drying step dries the conductive ink composition that has been applied to the substrate at temperature of 140 °C.

8. The method of claim 1, wherein the drying step dries the conductive ink composition that has been applied to the substrate with an air flow of 60 m³/min.

9. The method of claim 1, wherein the drying step has a duration in a range of 30 seconds to 60 seconds.

10. The method of claim 1, wherein the transporting and printing steps are accomplished using a continuous web-based process.

## Patentansprüche

1. Verfahren zum Herstellen eines elektrochemischen Sensors, welcher ein Substrat und wenigstens zwei Schichten, die auf das Substrat zu drucken sind, aufweist, wobei das Verfahren das Transportieren einer Lage des Substrats durch eine erste und eine zweite Druckstation, eine Trockenstation, die zwischen den Druckstationen angeordnet ist, und eine Kühlstation, die sich zwischen der Trockenstation und der zweiten Druckstation befindet; das Drucken einer Schicht aus leitender Farbe auf das Substrat, wenn es durch die erste Druckstation transportiert wird, indem eine leitende Farbzusammensetzung auf das Substrat aufgebracht wird, wobei die leitende Farbzusammensetzung aufweist:
Graphit;
Ruß;
ein Harz; und
wenigstens ein Lösemittel;
wobei ein Gewichtsverhältnis des Graphit zum Ruß in einem Bereich von
4:1 bis 1:4 ist; und
wobei ein Gewichtsverhältnis einer Summe aus Graphit und Ruß zu Harz in einem Bereich von 10:1 1 bis 1:1 1 ist;
Trocknen der ersten Schicht aus leitender Farbe auf dem Substrat in der Trockenstation und Kühlen des gedruckten Substrats in der Kühlstation vor dem Aufdrucken einer zweiten Schicht auf das Substrat, wenn es durch eine zweite Druckstation transportiert wird, aufweist.

2. Verfahren nach Anspruch 1, bei dem das Lösemittel in der leitenden Farbzusammensetzung einen Siedepunkt zwischen 120°C und 250°C hat.

3. Verfahren nach Anspruch 1, bei dem das Lösemittel in der leitenden Farbzusammensetzung Isophoron, Diacetonalkohol und Methoxypropoxypropanol umfaßt.

4. Verfahren nach Anspruch 1, bei dem das Harz in der leitenden Farbzusammensetzung ein Terpolymer ist, welches Vinylchlorid, Vinylacetat und Vinylalkohol umfaßt.

5. Verfahren nach Anspruch 1, bei dem das Verhältnis von Graphit zu Ruß in der leitenden Farbzusammensetzung 2.62:1 ist und das Verhältnis der Summe von Graphit und Ruß zu Harz 2.9:1 ist.

6. Verfahren nach Anspruch 1, bei dem eine Teilchengröße des Graphits in der leitenden Farbzusammensetzung 15 µm ist.

7. Verfahren nach Anspruch 1, bei dem der Trockenschritt die leitende Farbzusammensetzung, die auf das Substrat aufgegeben worden ist, bei einer Temperatur von 140°C trocknet.

8. Verfahren nach Anspruch 1, bei dem der Trockenschritt die leitende Farbzusammensetzung, die auf das Substrat aufgebracht worden ist, mit einem Luftdurchfluß von 60 m³/min trocknet.

9. Verfahren nach Anspruch 1, bei dem der Trockenschritt eine Dauer in einem Bereich von 30 Sekunden bis 60 Sekunden hat.

10. Verfahren nach Anspruch 1, bei dem die Transport- und Druckschritte bewerkstelligt werden, indem ein auf einer kontinuierlichen Lage basierender Prozeß verwendet wird.

## Revendications

1. Procédé de fabrication d'un capteur électrochimique comprenant un substrat et au moins deux couches devant être imprimées sur le substrat, le procédé comprenant le transport d'une bobine du substrat au-delà d'un premier et d'un second groupe d'impression, un groupe de séchage, situé entre lesdits groupes d'impression, et un groupe de refroidissement, situé entre le groupe de séchage et le second groupe d'impression ; l'impression d'une encre conductrice sur le substrat lors de son transport au-delà du premier groupe d'impression en appliquant une composition d'encre conductrice sur le substrat, la composition d'encre conductrice comprenant :
du graphite ;
du noir de carbone ;
une résine ; et
au moins un solvant ;
dans lequel un rapport massique du graphite au noir de carbone est dans la gamme allant de 4:1 à 1:4 ; et
dans lequel un rapport massique d'un ensemble graphite et noir de carbone à la résine est dans la gamme allant de 10:1 à 1:1;
le séchage de la première couche d'encre conductrice sur le substrat dans le groupe de séchage et le refroidissement du substrat imprimé dans le groupe de refroidissement avant impression d'une seconde couche sur le substrat lors de son transport au-delà du second groupe d'impression.

2. Procédé selon la revendication 1, dans lequel le solvant de la composition d'encre conductrice a un point d'ébullition compris entre 120 °C et 250 °C.

3. Procédé selon la revendication 1, dans lequel le solvant de la composition d'encre conductrice contient de l'isophorone, du diacétone alcool et un méthoxy propoxy propanol.

4. Procédé selon la revendication 1, dans lequel la résine de la composition d'encre conductrice est un terpolymère incluant du chlorure de vinyle, de l'acétate de vinyle et de l'alcool vinylique.

5. Procédé selon la revendication 1, dans lequel le rapport du graphite au noir de carbone dans la composition d'encre conductrice est de 2,62:1 et le rapport de la somme du graphite et du noir de carbone à la résine est de 2,9:1.

6. Procédé selon la revendication 1, dans lequel une taille de particule du graphite dans la composition d'encre conductrice est de 15 µm.

7. Procédé selon la revendication 1, dans lequel l'étape de séchage sèche la composition d'encre conductrice qui a été appliquée au substrat à une température de 140 °C.

8. Procédé selon la revendication 1, dans lequel l'étape de séchage sèche la composition d'encre conductrice qui a été appliquée au substrat par un flux d'air de 60 m³/min.

9. Procédé selon la revendication 1, dans lequel l'étape de séchage à une durée pouvant aller de 30 secondes à 60 secondes.

10. Procédé selon la revendication 1, dans lequel les étapes de transport et d'impression sont accomplies en utilisant un procédé continu à base de bobine.
